# EUROPEAN PATENT APPLICATION

(11) **EP 3 616 685 A1**
(43) Date of publication of application: **04.03.2020**
(21) Application number: 18792151.5
(22) Date of filing: 26.04.2018
(51) Int. Cl.: A61K 9/00, A61J 3/06, A61K 9/44

(54) **METHOD FOR DETERMINING AUTHENTICITY OF PHARMACEUTICAL COMPOSITION FOR ORAL ADMINISTRATION, PHARMACEUTICAL COMPOSITION FOR ORAL ADMINISTRATION, AND SYSTEM FOR DETERMINING AUTHENTICITY OF PHARMACEUTICAL COMPOSITION FOR ORAL ADMINISTRATION**

(30) Priority: 28.04.2017 JP 2017089190
(71) Applicant: Eisai R&D Management Co., Ltd., Tokyo 112-8088 (JP)
(72) Inventor: AOKI, Shigeru, Kakamigahara-shi Gifu 501-6195 (JP); TAMURA, Kazuhiko, Kakamigahara-shi Gifu 501-6195 (JP); UCHIYAMA, Jumpei, Kakamigahara-shi Gifu 501-6195 (JP)
(74) Representative: Hoffmann Eitle
(86) International application number: PCT/JP2018/016894
(87) International publication number: WO 2018/199201

(57) **Abstract**

Provided is a method for determining the genuineness or spuriousness of a pharmaceutical composition for oral administration, comprising irradiating a pharmaceutical composition for oral administration with infrared light, the pharmaceutical composition for oral administration being marked with a mark including an edible substance which is visually recognizable under white light irradiation as well as under infrared irradiation and a mark including an edible substance which is visually recognizable under white light irradiation and is not visually recognizable under infrared irradiation, and determining whether a visually recognizable mark visually recognized on the pharmaceutical composition for oral administration irradiated with the infrared light and a prepared reference mark match.

## Description

### Technical Field

**The** present invention relates to a medicine, and to a method for determining the genuineness or spuriousness of a pharmaceutical composition for oral administration, a pharmaceutical composition for oral administration, and a system for determining the genuineness or spuriousness of a pharmaceutical composition for oral administration.

### Background Art

Anti-counterfeit techniques for preventing counterfeit of bills, cash vouchers, and other products have been proposed (see for example Patent Documents 1 to 7). Patent Document 8 discloses an anti-counterfeit ink which appears dark by visible light irradiation and emits fluorescent light by ultraviolet irradiation, so that the ink can be visually recognized. Patent Document 9 discloses an anti-counterfeit ink which changes in color as the temperature changes.

In recent years, counterfeit medicines have been distributed (see for example Patent Document 10). The counterfeit medicine is also called a counterfeit drug. The counterfeit medicine may not include an active ingredient or may include a harmful substance. Therefore, intake of one of these counterfeit medicines may jeopardize the chance of treatment or give rise to a health problem. However, the anti-counterfeit inks disclosed in Patent Documents 8 and 9 are not edible. Therefore, medicines themselves cannot be printed with marks using any of the anti-counterfeit inks as disclosed in Patent Documents 8 and 9.

**Therefore,** according to a proposed method, a hologram is attached to a wrapping material of a medicine or a wrapping material is printed with an identification code which can be identified only by an actual manufacturer, so that the authenticity can be determined. However, the medicine in the wrapping material can be exchanged from the genuine medicine to a counterfeit medicine for example by the manufacturer of the counterfeit medicine. To counter the problem, according to another proposed method, the wrapping material is sealed, so that the presence/absence of alteration can be identified.

### Citation List

### Patent Documents

Patent Document 1: Patent Publication JP-A-S58-134782
Patent Document 2: Patent Publication JP-A-S63-92486
Patent Document 3: Patent Publication JP-A-H02-167771
Patent Document 4: Patent Publication JP-A-H03-154187
Patent Document 5: Patent Publication JP-A-2004-348539
Patent Document 6: Patent Publication JP-A-2006-205500
Patent Document 7: Patent Publication JP-A-2011-178009
Patent Document 8: Patent Publication JP-A-2010-6938
Patent Document 9: Patent Publication JP-A-2008-189879
Patent Document 10: Patent Publication JP-A-2006-89741

### Summary

### Technical Problem

A pharmaceutical composition for oral administration, in the form of a tablet or a capsule in particular, is printed with a mark using an edible ink or provided with a mark by marking using ultraviolet (UV) laser light irradiation in order to identify the product. The mark is printed by transfer printing using for example an ink and a rubber roller or by multi-color ink-jet printing.

Examples of the mark include a manufacturing company's name, the logo of the manufacturing company, a product name, and a product number. The marks for identifying a pharmaceutical composition for oral administration have never been used for determining the genuineness or spuriousness of the pharmaceutical composition for oral administration. Edible pigments or inks available for determining the genuineness or spuriousness of a pharmaceutical composition for oral administration have been unknown.

Therefore, it is an object of the present invention to provide a method for determining the genuineness or spuriousness of a pharmaceutical composition for oral administration to make it possible to determine the genuineness or spuriousness of a pharmaceutical composition for oral administration, a pharmaceutical composition for oral administration, and a system for determining the genuineness or spuriousness of a pharmaceutical composition for oral administration.

### Solution to Problem

According to an embodiment of the present invention, provided is a method for determining the genuineness or spuriousness of a pharmaceutical composition for oral administration, comprising irradiating a pharmaceutical composition for oral administration with infrared light, the pharmaceutical composition for oral administration being marked with a mark including an edible substance which is visually recognizable under white light irradiation as well as under infrared irradiation and a mark including an edible substance which is visually recognizable under white light irradiation and is not visually recognizable under infrared irradiation, and determining whether the visually recognizable mark visually recognized on the pharmaceutical composition for oral administration irradiated with the infrared light and a prepared reference mark match.

In the method for determining the genuineness or spuriousness of a pharmaceutical composition for oral administration, the infrared light may have a wavelength within a wavelength band range which includes at least a part of the range of 700 nm or more and 900 nm or Iress.

In the method for determining the genuineness or spuriousness of a pharmaceutical composition for oral administration, the wavelength band range of the infrared light may be a range of 900 nm or more.

In the method for determining the genuineness or spuriousness of a pharmaceutical composition for oral administration, the pharmaceutical composition for oral administration may include titanium oxide (TiO₂) and the mark including an edible substance which is visually recognizable under infrared irradiation may be marked by laser irradiation.

In the method for determining the genuineness or spuriousness of a pharmaceutical composition for oral administration, the mark including the edible substance which is visually recognizable under infrared irradiation may be laser-printed using a pigment including yellow iron sesquioxide.

In the method for determining the genuineness or spuriousness of a pharmaceutical composition for oral administration, the mark including the edible substance which is visually recognizable under infrared irradiation may be printed using a pigment including at least one of edible charcoal and black triiron tetraoxide (Fe₃O₄).

In the method for determining the genuineness or spuriousness of a pharmaceutical composition for oral administration, the mark including the edible substance which is visually recognizable under infrared irradiation may be printed using a pigment including at least one selected from the group consisting of red iron oxide (Fe₂O₃), sodium copper chlorophyllin, indigocarmine, and black titanium oxide when the wavelength band range of the infrared light includes at least a part of the range of 700 nm or more and 900 nm or less.

In the method for determining the genuineness or spuriousness of a pharmaceutical composition for oral administration, the mark including the edible substance which is not visually recognizable under infrared irradiation may be printed using a dye.

In the method for determining the genuineness or spuriousness of a pharmaceutical composition for oral administration, the dye may include a tar dye.

In the method for determining the genuineness or spuriousness of a pharmaceutical composition for oral administration, the tar dye may include at least one selected from the group consisting of erythrosine, New Coccine, tartrazine, brilliant blue FCF, and sunset yellow FCF.

In the method for determining the genuineness or spuriousness of a pharmaceutical composition for oral administration, the mark including the edible substance which is not visually recognizable under infrared irradiation may be printed using a pigment including a tar dye aluminum lake.

In the method for determining the genuineness or spuriousness of a pharmaceutical composition for oral administration, the mark including the edible substance which is not visually recognized under infrared irradiation may be printed using a pigment including red iron oxide (Fe₂O₃) when the wavelength band range of the infrared light is a range of 900 nm or more.

In the determining step of the method for determining the genuineness or spuriousness of a pharmaceutical composition for oral administration, the reference mark may correspond to an identifying number or an identifying code for the pharmaceutical composition for oral administration.

In the method for determining the genuineness or spuriousness of a pharmaceutical composition for oral administration, the identifying number or the identifying code may be marked on the pharmaceutical composition for oral administration.

In the method for determining the genuineness or spuriousness of a pharmaceutical composition for oral administration, the pharmaceutical composition for oral administration may be in a tablet form or a capsule form.

In the method for determining the genuineness or spuriousness of a pharmaceutical composition for oral administration, the pharmaceutical composition for oral administration may be in a tablet form.

According to the embodiment of the present invention, provided is a pharmaceutical composition for oral administration marked with a mark including an edible substance which is visually recognizable under white light irradiation as well as under infrared irradiation and a mark including an edible substance which is visually recognizable under white light irradiation and is not visually recognizable under infrared irradiation, the pharmaceutical composition for oral administration is able to be authenticated by determining whether the visually recognizable mark visually recognized on the pharmaceutical composition for oral administration and a predetermined reference mark match.

In the pharmaceutical composition for oral administration, the infrared light may have a wavelength within a wavelength band range which includes at least a part of the range of 700 nm or more and 900 nm or less.

In the pharmaceutical composition for oral administration, the wavelength band range of the infrared light may be a range of 900 nm or more.

The pharmaceutical composition for oral administration may include titanium oxide (TiO₂) and may be marked by laser irradiation with the mark including the edible substance which is visually recognizable under infrared irradiation.

In the pharmaceutical composition for oral administration, the mark including the edible substance which is visually recognizable under infrared irradiation may be laser-printed using a pigment including yellow iron sesquioxide.

In the pharmaceutical composition for oral administration, the mark including the edible substance which is visually recognizable under infrared irradiation may be printed using a pigment including at least one of edible charcoal and black triiron tetraoxide (Fe₃O₄).

In the pharmaceutical composition for oral administration, the mark including the edible substance which is visually recognizable under infrared irradiation may be printed using a pigment including at least one selected from the group consisting of red iron oxide (Fe₂O₃), sodium copper chlorophyllin, and indigocarmine, when the wavelength band range of the infrared light includes at least a part of the range of 700 nm or more and 900 nm or less.

In the pharmaceutical composition for oral administration, the mark including the edible substance which is not visually recognizable under infrared irradiation may be printed using a dye.

In the pharmaceutical composition for oral administration, the dye may include a tar dye.

In the pharmaceutical composition for oral administration, the tar dye may include at least one selected from the group consisting of erythrosine, New Coccine, tartrazine, brilliant blue FCF, and sunset yellow FCF.

In the pharmaceutical composition for oral administration, the mark including the edible substance which is not visually recognizable under infrared irradiation may be printed using a pigment including a tar dye aluminum lake.

In the pharmaceutical composition for oral administration, the mark including the edible substance which is not visually recognizable under infrared irradiation may be printed using a pigment including red iron oxide (Fe₂O₃) when the wavelength band range of the infrared light is a range of 900 nm or more.

In the determination about the pharmaceutical composition for oral administration, the reference mark may correspond to an identifying number or an identifying code for the pharmaceutical composition for oral administration.

The pharmaceutical composition for oral administration may be marked with the identifying number or the identifying code.

The pharmaceutical composition for oral administration may be in a tablet form or a capsule form.

The pharmaceutical composition for oral administration may be in a tablet form.

According to the embodiment of the present invention, provided is a system for determining the genuineness or spuriousness of a pharmaceutical composition for oral administration, comprising an irradiating device configured to irradiate a pharmaceutical composition for oral administration with infrared light, the pharmaceutical composition for oral administration being marked with a mark including an edible substance which is visually recognizable under white light irradiation as well as under infrared irradiation and a mark including an edible substance which is visually recognizable under white light irradiation and is not visually recognizable under infrared irradiation, and a determining unit configured to determine whether the visually recognizable mark visually recognized on the pharmaceutical composition for oral administration irradiated with the infrared light and a prepared reference mark match.

In the system for determining the genuineness or spuriousness of a pharmaceutical composition for oral administration, the wavelength band range of the infrared light may include at least a part of the range of 700 nm or more and 900 nm or less.

In the system for determining the genuineness or spuriousness of a pharmaceutical composition for oral administration, the wavelength band range of the infrared light may be a range of 900 nm or more.

In the system for determining the genuineness or spuriousness of a pharmaceutical composition for oral administration, the pharmaceutical composition for oral administration may include titanium oxide (TiO₂) and may be marked by laser irradiation with the mark including an edible substance which is visually recognizable under infrared irradiation.

In the system for determining the genuineness or spuriousness of a pharmaceutical composition for oral administration, the mark including the edible substance which is visually recognizable under infrared irradiation may be laser-printed using a pigment including yellow iron sesquioxide.

In the system for determining the genuineness or spuriousness of a pharmaceutical composition for oral administration, the mark including the edible substance which is visually recognizable under infrared irradiation may be printed using a pigment including at least one of edible charcoal and black triiron tetraoxide (Fe₃O₄).

In the system for determining the genuineness or spuriousness of a pharmaceutical composition for oral administration, the mark including the edible substance which is visually recognizable under infrared irradiation may be printed using a pigment including at least one selected from the group consisting of red iron oxide (Fe₂O₃), sodium copper chlorophyllin, and indigocarmine when the wavelength band range of the infrared light includes at least a part of the range of 700 nm or more and 900 nm or less.

In the system for determining the genuineness or spuriousness of a pharmaceutical composition for oral administration, the mark including the edible substance which is not visually recognizable under infrared irradiation may be printed using a dye.

In the system for determining the genuineness or spuriousness of a pharmaceutical composition for oral administration, the dye may include a tar dye.

In the system for determining the genuineness or spuriousness of a pharmaceutical composition for oral administration, the tar dye may include at least one selected from the group consisting of erythrosine, New Coccine, tartrazine, brilliant blue FCF, and sunset yellow FCF.

In the system for determining the genuineness or spuriousness of a pharmaceutical composition for oral administration, the mark including the edible substance which is not visually recognizable under infrared irradiation may be printed using a pigment including a tar dye aluminum lake.

In the system for determining the genuineness or spuriousness of a pharmaceutical composition for oral administration, the mark including the edible substance which is not visually recognizable under infrared irradiation may be printed using a pigment including red iron oxide (Fe₂O₃) when the wavelength band range of the infrared light is a range of 900 nm or more.

In the determination by the system for determining the genuineness or spuriousness of a pharmaceutical composition for oral administration, the reference mark may correspond to an identifying number or an identifying code for the pharmaceutical composition for oral administration.

In the system for determining the genuineness or spuriousness of a pharmaceutical composition for oral administration, the identifying number or the identifying code is marked on the pharmaceutical composition for oral administration.

In the system for determining the genuineness or spuriousness of a pharmaceutical composition for oral administration, the pharmaceutical composition for oral administration may be in a tablet form or a capsule form.

In the system for determining the genuineness or spuriousness of a pharmaceutical composition for oral administration, the pharmaceutical composition for oral administration may be in a tablet form.

### Advantageous Effects of Invention

According to the present invention, a method for determining the genuineness or spuriousness of a pharmaceutical composition for oral administration, the authenticity or counterfeit nature of which can be distinguished, a pharmaceutical composition for oral administration, and a system for determining the genuineness or spuriousness of a pharmaceutical composition for oral administration can be provided.

### Brief Description of Drawings

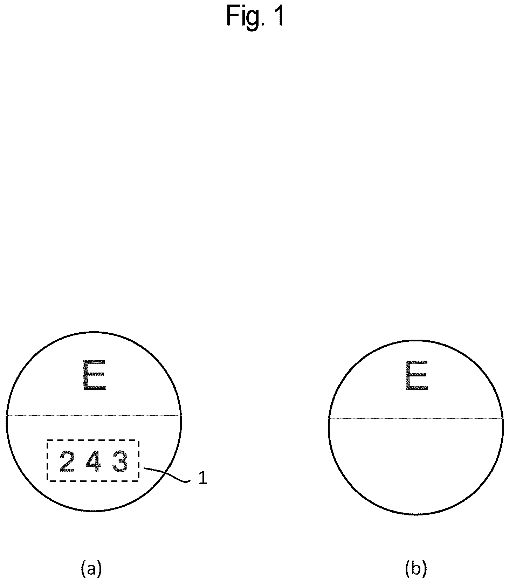

Fig. 1 schematically shows a pharmaceutical composition for oral administration marked with marks according to an embodiment of the invention. Fig. 1(a) is a schematic view of the pharmaceutical composition for oral administration observed under white light, and Fig. 1(b) is a schematic view of the pharmaceutical composition for oral administration observed under infrared light having a wavelength within the range of 700 nm or more and 1700 nm or less.

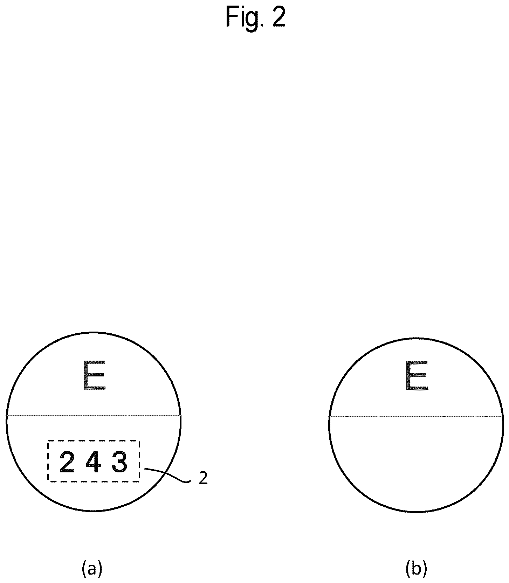

Fig. 2 schematically shows the pharmaceutical composition for oral administration marked with the marks according to the embodiment. Fig. 2(a) is a schematic view of the pharmaceutical composition for oral administration observed under white light, and Fig. 2(b) is a schematic view of the pharmaceutical composition for oral administration observed under infrared light having a wavelength within the range of 900 nm or more and 1700 nm or less.

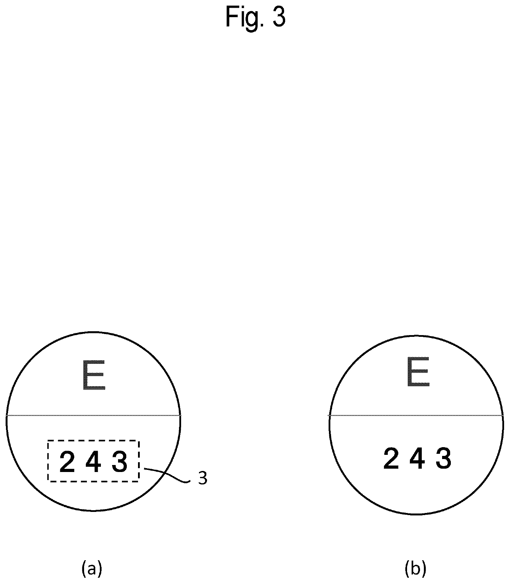

Fig. 3 schematically shows a pharmaceutical composition for oral administration marked with marks according to a reference example. Fig. 3(a) is a schematic view of the pharmaceutical composition for oral administration observed under white light, and Fig. 3(b) is a schematic view of the pharmaceutical composition for oral administration observed under infrared light having a wavelength within the range of 700 nm or more and 1700 nm or less.

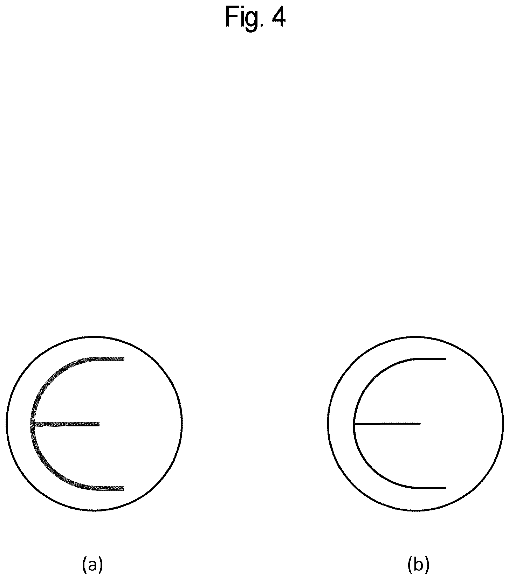

Fig. 4 schematically shows a pharmaceutical composition for oral administration marked with a mark according to the embodiment. Fig. 4(a) is a schematic view of the pharmaceutical composition for oral administration observed under white light, and Fig. 4(b) is a schematic view of the pharmaceutical composition for oral administration observed under infrared light having a wavelength in the range of 700 nm or more and 1700 nm or less.

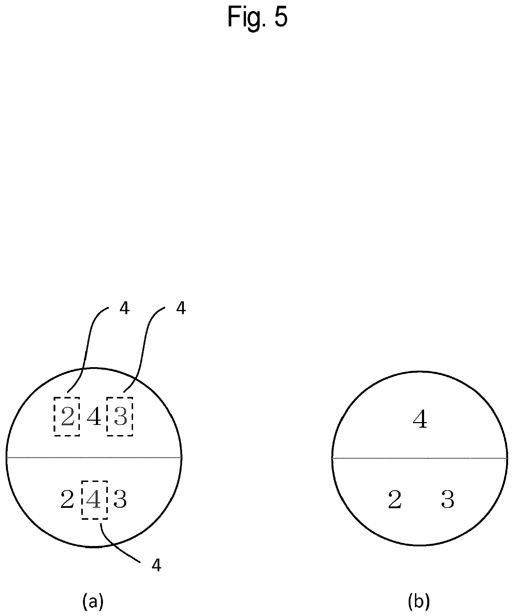

Fig. 5 schematically shows a pharmaceutical composition for oral administration marked with marks according to the embodiment. Fig. 5(a) is a schematic view of the pharmaceutical composition for oral administration observed under white light, and Fig. 5(b) is a schematic view of the pharmaceutical composition for oral administration observed under infrared light having a wavelength within the range of 700 nm or more and 1700 nm or less.

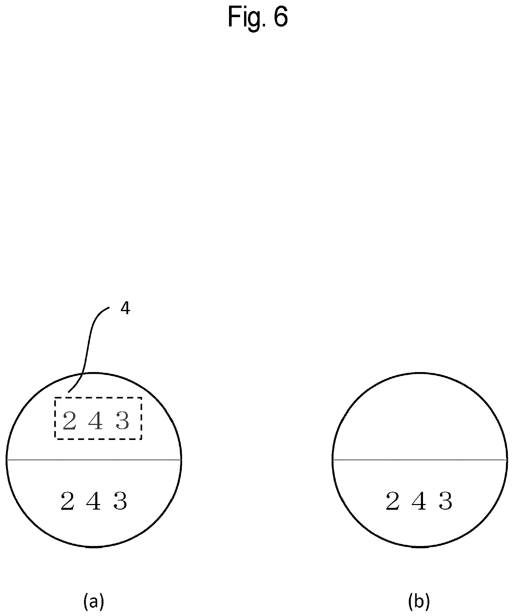

Fig. 6 schematically shows a pharmaceutical composition for oral administration marked with marks according to the embodiment. Fig. 6(a) is a schematic view of the pharmaceutical composition for oral administration observed under white light, and Fig. 6(b) is a schematic view of the pharmaceutical composition for oral administration observed under infrared light having a wavelength within the range of 700 nm or more and 1700 nm or less.

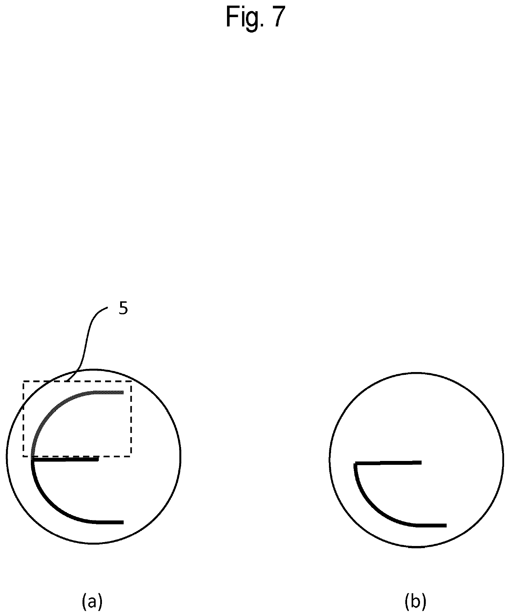

Fig. 7 schematically shows a pharmaceutical composition for oral administration marked with a mark according to the embodiment. Fig. 7(a) is a schematic view of the pharmaceutical composition for oral administration observed under white light, and Fig. 7(b) is a schematic view of the pharmaceutical composition for oral administration observed under infrared light having a wavelength within the range of 700 nm or more and 1700 nm or less.

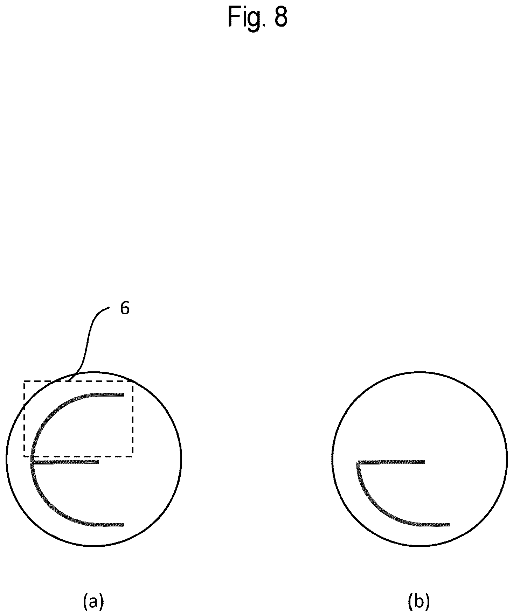

Fig. 8 schematically shows a pharmaceutical composition for oral administration marked with a mark according to the embodiment. Fig. 8(a) is a schematic view of the pharmaceutical composition for oral administration observed under white light, and Fig. 8(b) is a schematic view of the pharmaceutical composition for oral administration observed under infrared light having a wavelength within the range of 700 nm or more and 900 nm or less.

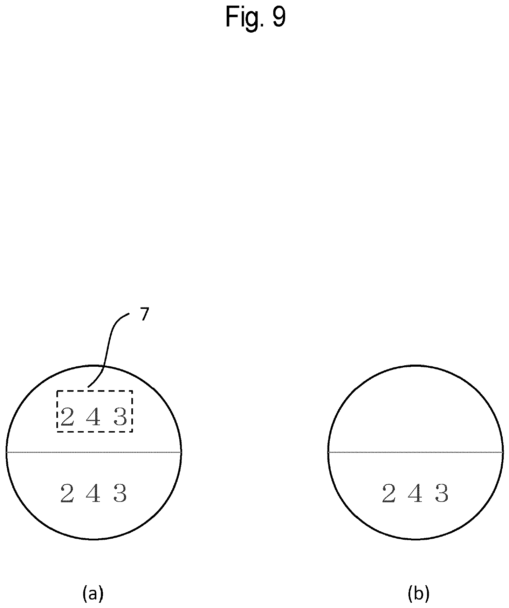

Fig. 9 schematically shows a pharmaceutical composition for oral administration marked with marks according to the embodiment. Fig. 9(a) is a schematic view of the pharmaceutical composition for oral administration observed under white light, and Fig. 9(b) is a schematic view of the pharmaceutical composition for oral administration observed under infrared light having a wavelength within the range of 700 nm or more and 900 nm or less.

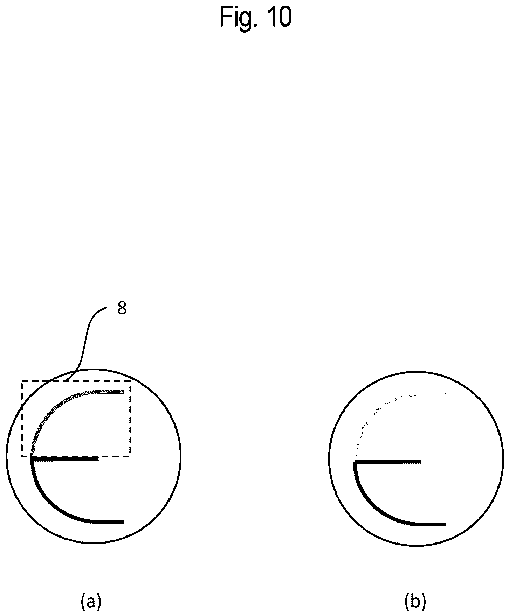

Fig. 10 schematically shows a pharmaceutical composition for oral administration marked with a mark according to the embodiment. Fig. 10(a) is a schematic view of the pharmaceutical composition for oral administration observed under white light, and Fig. 10(b) is a schematic view of the pharmaceutical composition for oral administration observed under infrared light having a wavelength within the range of 700 nm or more and 900 nm or less.

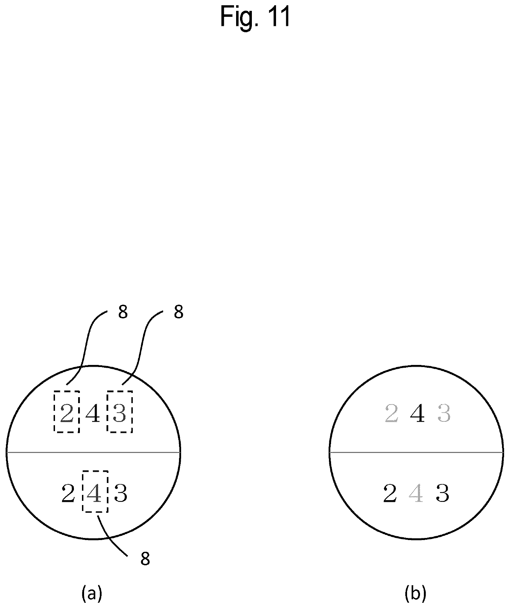

Fig. 11 schematically shows a pharmaceutical composition for oral administration marked with marks according to the embodiment. Fig. 11(a) is a schematic view of the pharmaceutical composition for oral administration observed under white light, and Fig. 11(b) is a schematic view of the pharmaceutical composition for oral administration observed under infrared light having a wavelength within the range of 700 nm or more and 900 nm or less.

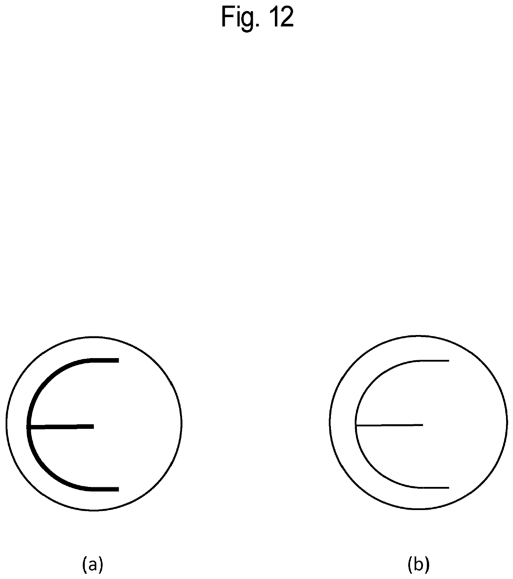

Fig. 12 schematically shows a pharmaceutical composition for oral administration marked with a mark according to the embodiment. Fig. 12(a) is a schematic view of the pharmaceutical composition for oral administration observed under white light, and Fig. 12(b) is a schematic view of the pharmaceutical composition for oral administration observed under infrared light having a wavelength within the range of 700 nm or more and 1700 nm or less.

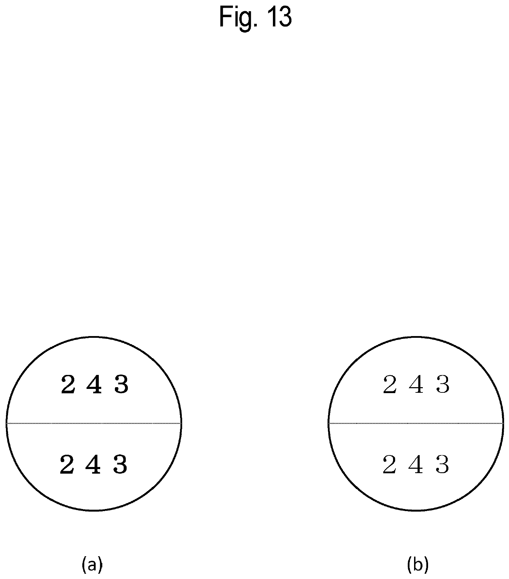

Fig. 13 schematically shows a pharmaceutical composition for oral administration marked with marks according to the embodiment. Fig. 13(a) is a schematic view of the pharmaceutical composition for oral administration observed under white light, and Fig. 13(b) is a schematic view of the pharmaceutical composition for oral administration observed under infrared light having a wavelength within the range of 700 nm or more and 1700 nm or less.

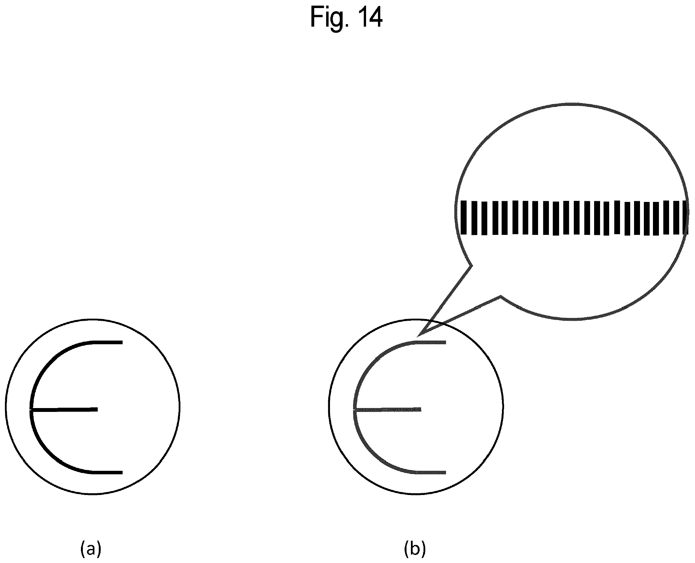

Fig. 14 schematically shows a pharmaceutical composition for oral administration marked with a mark according to the embodiment. Fig. 14(a) is a schematic view of the pharmaceutical composition for oral administration observed under white light, and Fig. 14(b) is a schematic view of the pharmaceutical composition for oral administration observed under infrared light having a wavelength within the range of 700 nm or more and 1700 nm or less.

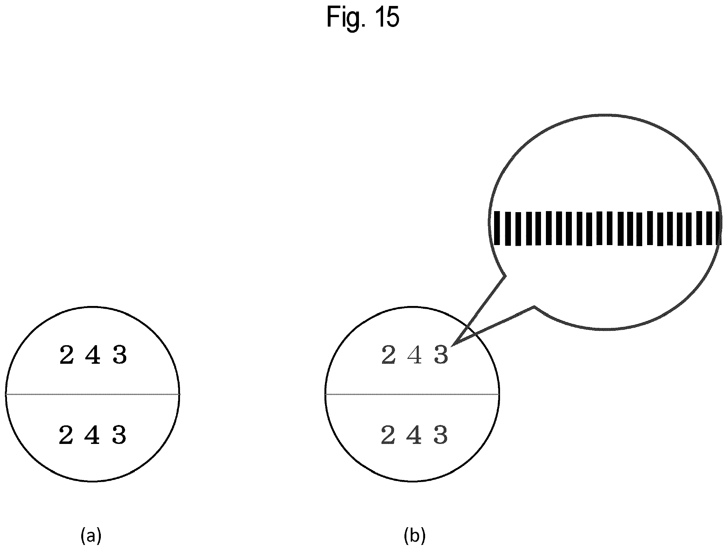

Fig. 15 schematically shows a pharmaceutical composition for oral administration marked with marks according to the embodiment. Fig. 15(a) is a schematic view of the pharmaceutical composition for oral administration observed under white light, and Fig. 15(b) is a schematic view of the pharmaceutical composition for oral administration observed under infrared light having a wavelength within the range of 700 nm or more and 1700 nm or less.

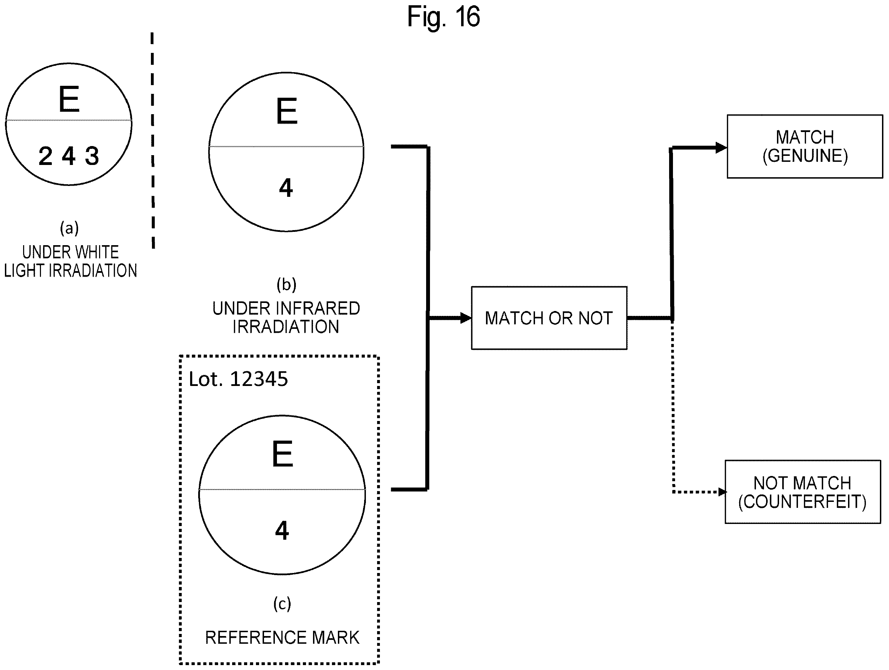

Fig. 16 is a schematic diagram for illustrating an example of the procedure of a method for determining the genuineness or spuriousness of a pharmaceutical composition for oral administration according to the embodiment.

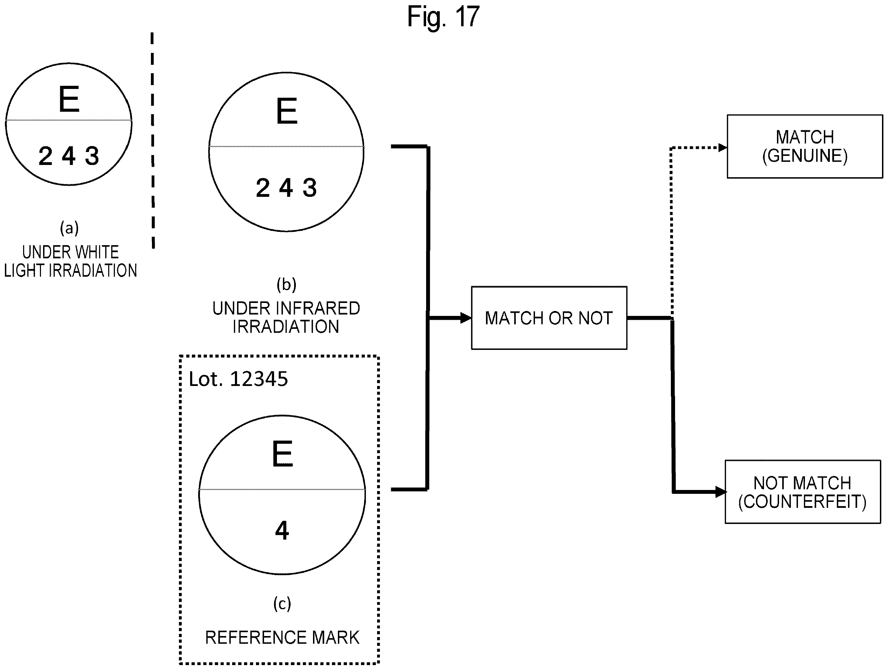

Fig. 17 is a schematic diagram for illustrating an example of the procedure of a method for determining the genuineness or spuriousness of a pharmaceutical composition for oral administration according to the embodiment.

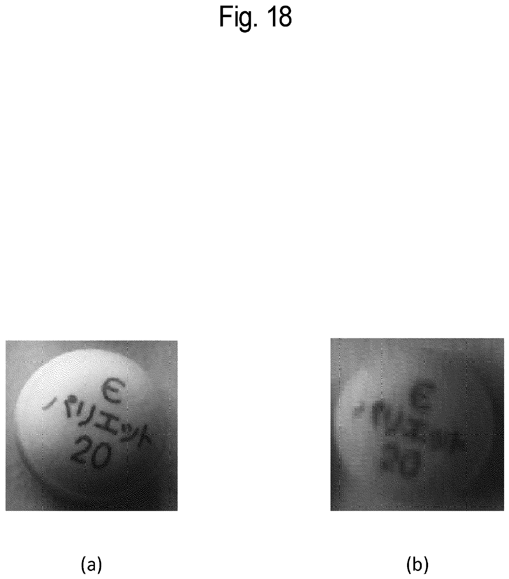

Fig. 18 is photographed data on a tablet marked with marks according to Example 1. Fig. 18(a) is photographed data on the tablet captured by a digital camera, and Fig. 18(b) is photographed data on the tablet captured by a near infrared camera under sunlight.

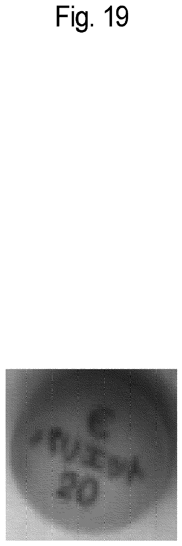

Fig. 19 is photographed data on the tablet marked with marks according to Example 1 captured by a near infrared camera when the tablet was irradiated with near infrared light having a wavelength band range within the range of 800 nm or more and 1000 nm or less.

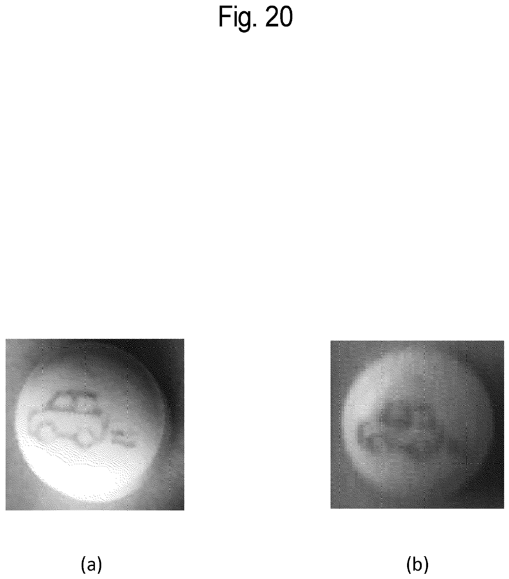

Fig. 20 is photographed data on a tablet marked with a mark according to Example 2. Fig. 20(a) is photographed data on the tablet captured by a digital camera, and Fig. 20(b) is photographed data on the tablet captured by a near infrared camera under sunlight.

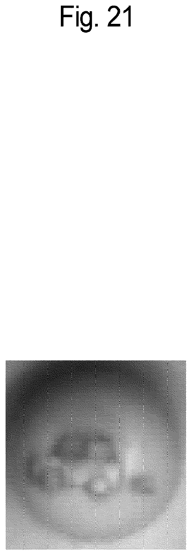

Fig. 21 is photographed data on a tablet marked with a mark according to Example 2 captured by a near infrared camera when the tablet was irradiated with near infrared light having a wavelength within a wavelength band range of 800 nm or more and 1000 nm or less.

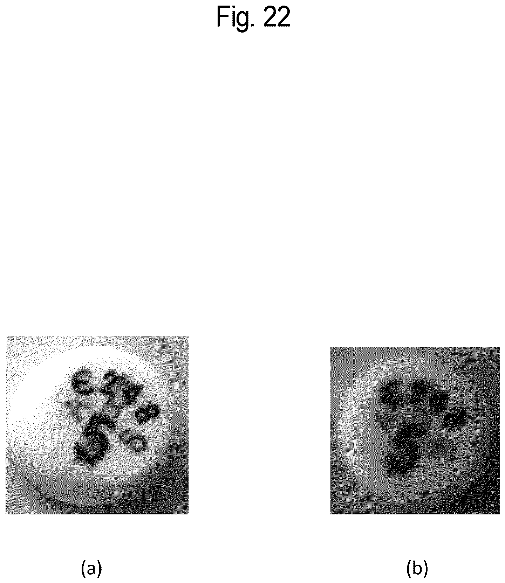

Fig. 22 is photographed data on a tablet marked with marks according to Example 3. Fig. 22(a) is photographed data on the tablet captured by a digital camera, and Fig. 22(b) is photographed data on the tablet captured by a near infrared camera under sunlight.

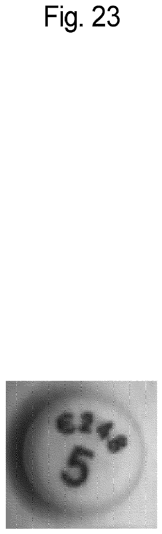

Fig. 23 is photographed data on a tablet marked with the marks according to Example 3 captured by a near infrared camera when the tablet was irradiated with near infrared light having a wavelength within a wavelength band range of 800 nm or more and 1000 nm or less.

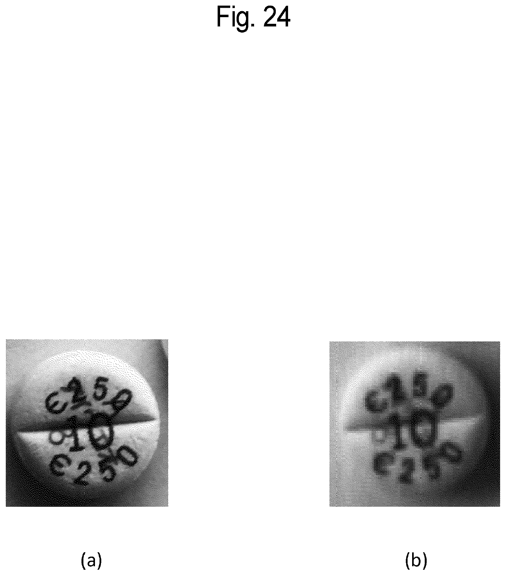

Fig. 24 is photographed data on a tablet marked with marks according to Example 4. Fig. 24(a) is photographed data on the tablet captured by a digital camera, and Fig. 24(b) is photographed data on the tablet captured by a near infrared camera under sunlight.

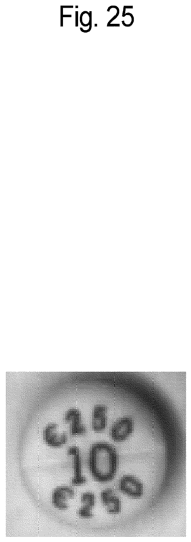

Fig. 25 is photographed data on a tablet marked with marks according to Example 4 captured by a near infrared camera when the tablet was irradiated with near infrared light having a wavelength within a wavelength band range of 800 nm or more and 1000 nm or less.

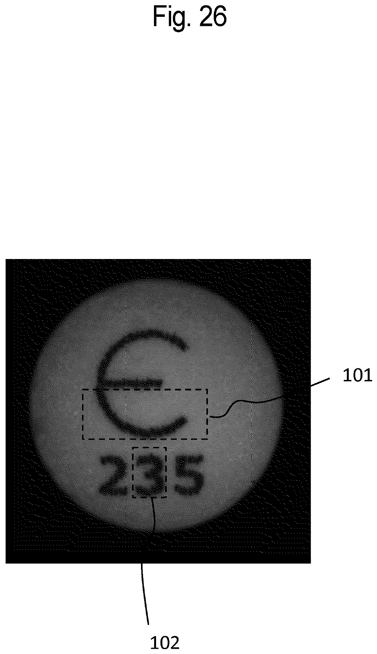

Fig. 26 is photographed data on the tablet marked with marks according to Example 5 captured by a digital camera.

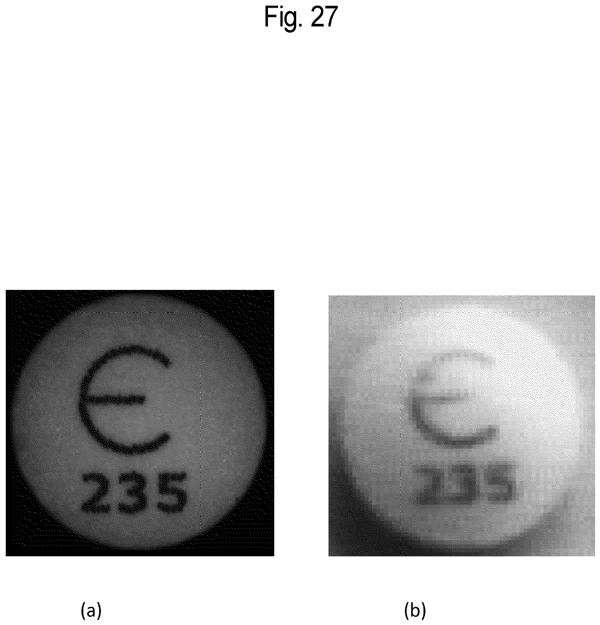

Fig. 27 is photographed data on a tablet marked with marks according to Example 5. Fig. 27(a) is photographed data on the tablet captured by a digital camera, and Fig. 27(b) is photographed data on the tablet captured by a near infrared camera under sunlight.

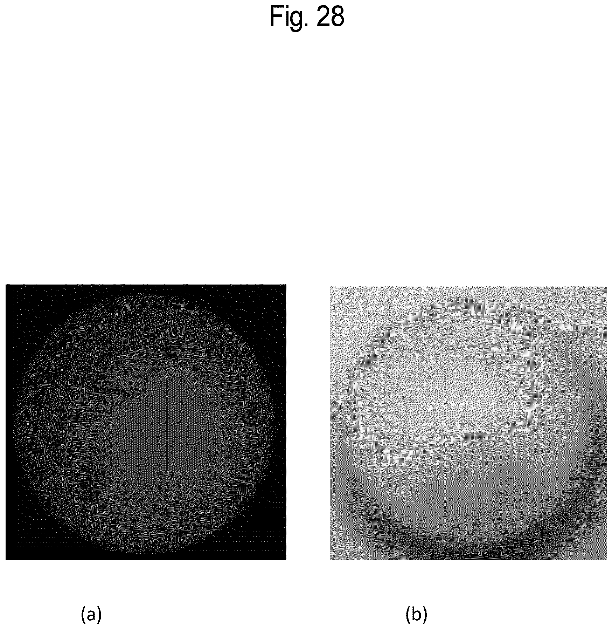

Fig. 28 is photographed data on the tablet marked with marks according to Example 5. Fig. 28(a) is photographed data on the tablet captured by a near infrared camera when the tablet was irradiated with near infrared light having a wavelength within a wavelength band range of 750 nm or more and 950 nm or less and Fig. 28(b) is photographed data on the tablet captured by a near infrared camera when the tablet was irradiated with near infrared light having a wavelength within a wavelength band range of 800 nm or more and 1000 nm or less.

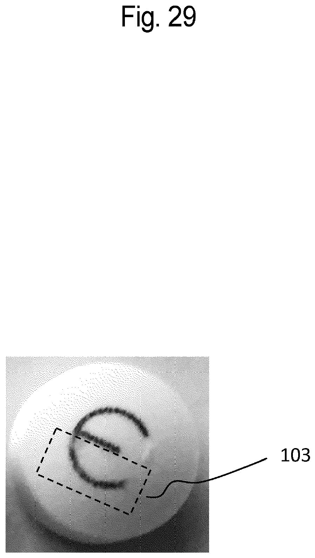

Fig. 29 is photographed data on a tablet marked with a mark according to Example 6 captured by a digital camera.

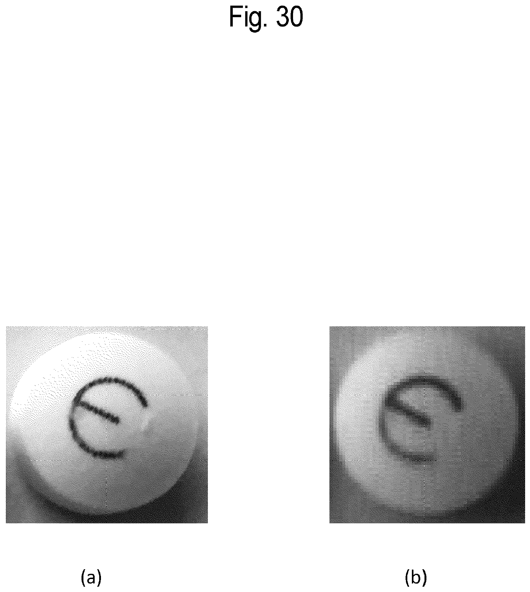

Fig. 30 is photographed data on the tablet marked with the mark according to Example 6. Fig. 30(a) is photographed data on the tablet captured by a digital camera, and Fig. 30(b) is photographed data on the tablet captured by a near infrared camera under sunlight.

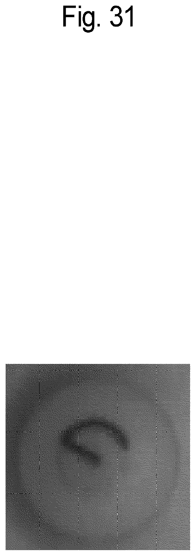

Fig. 31 is photographed data on the tablet marked with the mark according to Example 6 captured by a near infrared camera when the tablet is irradiated with near infrared light having a wavelength within a wavelength band range of 900 nm or more and 1000 nm or less.

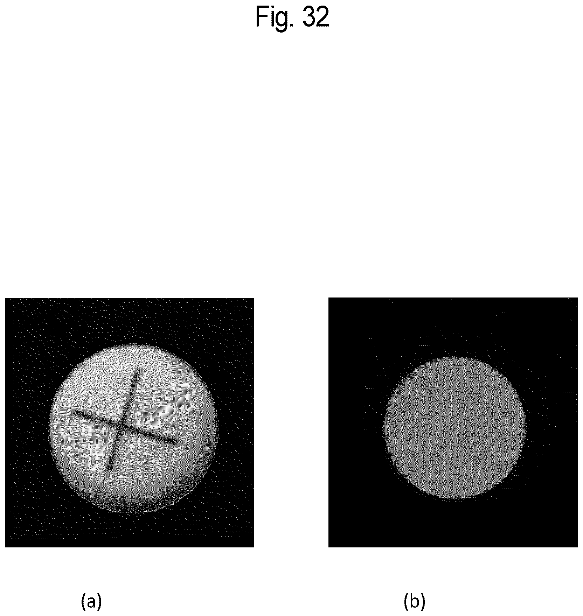

Fig. 32 is photographed data on a tablet marked with a mark according to a reference example. Fig. 32(a) is photographed data on the tablet captured by a digital camera, and Fig. 32(b) is photographed data on the tablet captured by a near infrared camera when the tablet is irradiated with near infrared light having a wavelength within a wavelength band range of 750 nm or more and 950 nm or less.

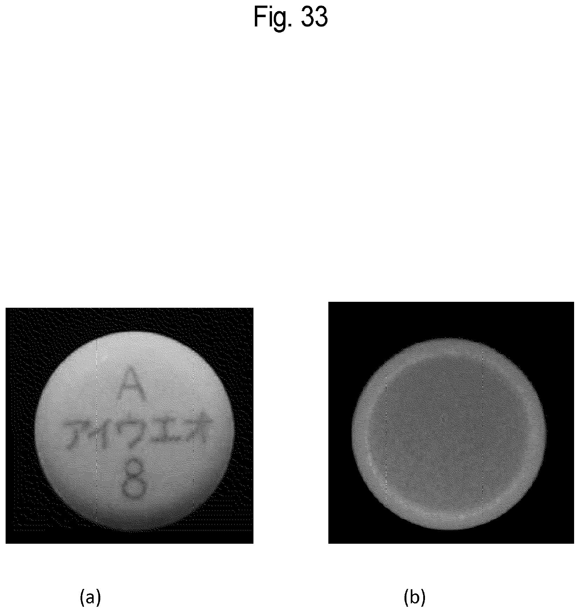

Fig. 33 is photographed data on the tablet marked with marks according to Example 7. Fig. 33(a) is photographed data on the tablet captured by a digital camera, and Fig. 33(b) is photographed data on the tablet captured by a near infrared camera when the tablet is irradiated with near infrared light having a wavelength within a wavelength band range of 750 nm or more and 950 nm or less.

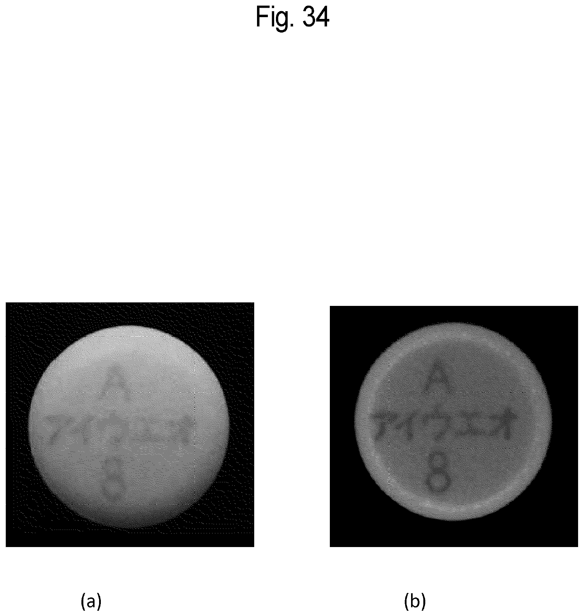

Fig. 34 is photographed data on the tablet marked with the marks according to Example 7. Fig. 34(a) is photographed data on the tablet captured by a digital camera, and Fig. 34(b) is photographed data on the tablet captured by a near infrared camera when the tablet was irradiated with near infrared light having a wavelength within a wavelength band range of 750 nm or more and 950 nm or less.

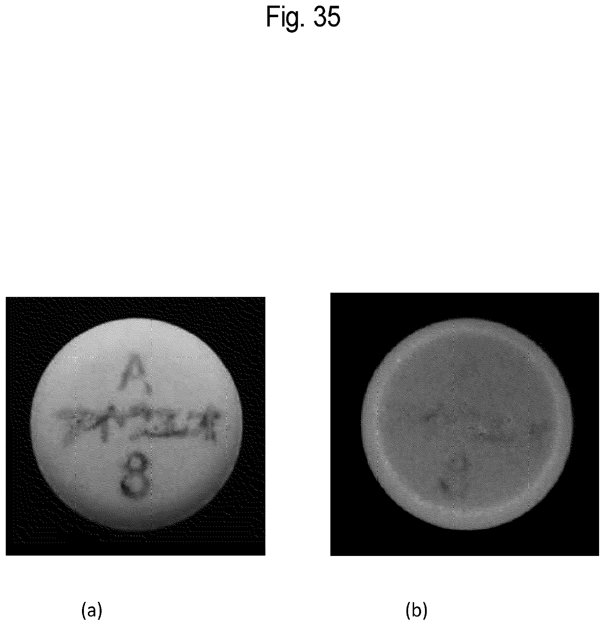

Fig. 35 is photographed data on the tablet marked with the marks according to Example 7. Fig. 35(a) is photographed data on the tablet captured by a digital camera, and Fig. 35(b) is photographed data on the tablet captured by a near infrared camera when the tablet is irradiated with near infrared light having a wavelength within a wavelength band range of 750 nm or more and 950 nm or less.

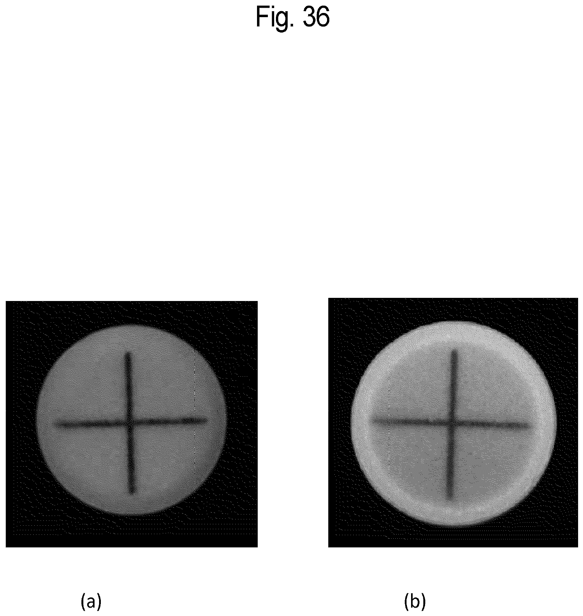

Fig. 36 is photographed data on a tablet marked with a mark according to Example 8. Fig. 36(a) is photographed data on the tablet captured by a digital camera, and Fig. 36(b) is photographed data on the tablet captured by a near infrared camera when the tablet is irradiated with near infrared light having a wavelength within a wavelength band range of 750 nm or more and 950 nm or less.

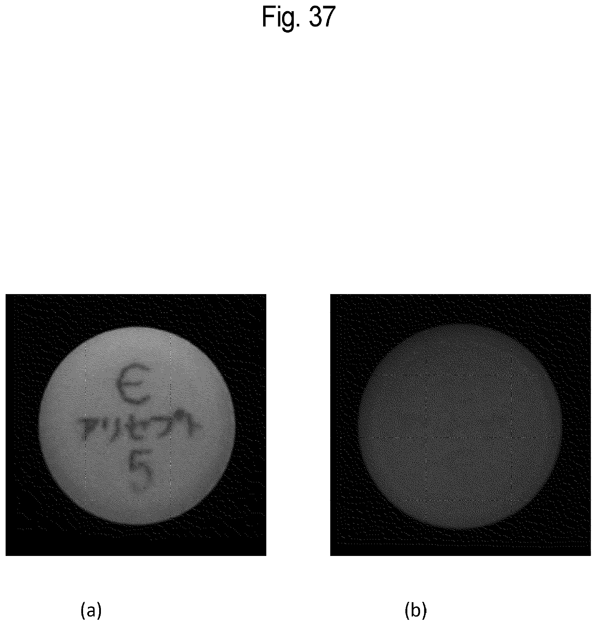

Fig. 37 is photographed data on the tablet marked with the marks according to Example 8. Fig. 37(a) is photographed data on the tablet captured by a digital camera, and Fig. 37(b) is photographed data on the tablet captured by a near infrared camera when the tablet was irradiated with near infrared light having a wavelength within a wavelength band range of 750 nm or more and 950 nm or less.

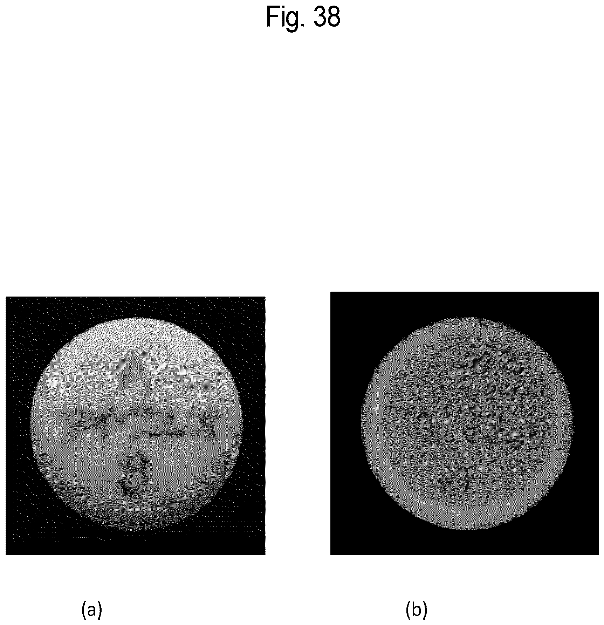

Fig. 38 is photographed data on a tablet marked with a mark according to Example 9. Fig. 38(a) is photographed data on the tablet captured by a digital camera, and Fig. 38(b) is photographed data on the tablet captured by a near infrared camera when the tablet was irradiated with near infrared light within a wavelength band range of 750 nm or more and 950 nm or less.

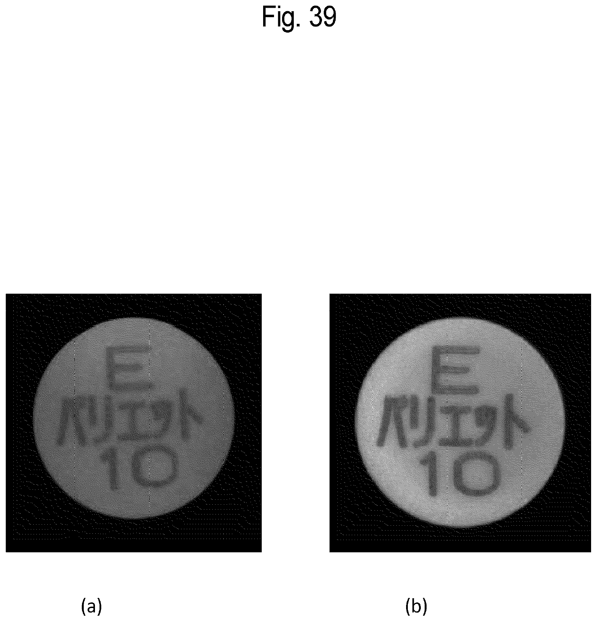

Fig. 39 is photographed data on a tablet marked with marks according to Example 10. Fig. 39(a) is photographed data on the tablet captured by a digital camera, and Fig. 39(b) is photographed data on the tablet captured by a near infrared camera when the tablet was irradiated with near infrared light having a wavelength within a wavelength band range of 750 nm or more and 950 nm or less.

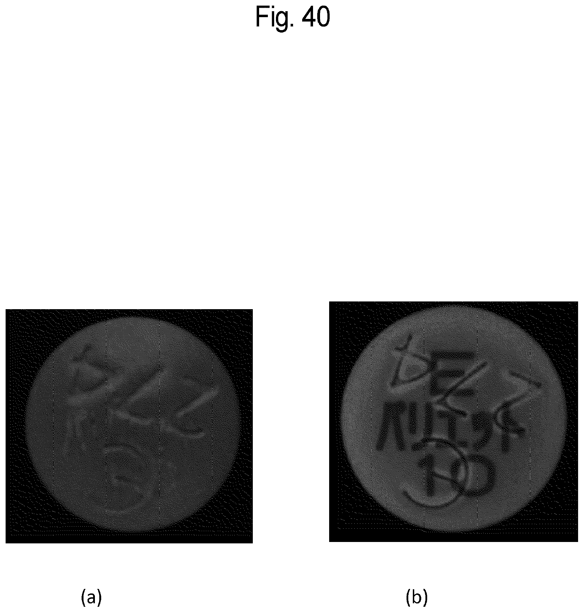

Fig. 40 is photographed data on a tablet marked with marks according to Example 10. Fig. 40(a) is photographed data on the tablet captured by a digital camera, and Fig. 40(b) is photographed data on the tablet captured by a near infrared camera when the tablet was irradiated with near infrared light having a wavelength within a wavelength band range of 750 nm or more and 950 nm or less.

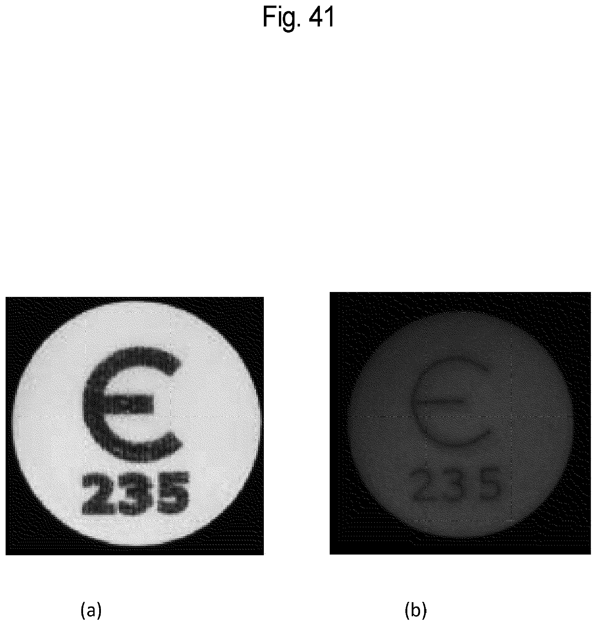

Fig. 41 is photographed data on a tablet marked with marks according to Example 11. Fig. 41(a) is photographed data on the tablet captured by a digital camera, and Fig. 41(b) is photographed data on the tablet captured by a near infrared camera when the tablet was irradiated with near infrared light having a wavelength within a wavelength band range of 750 nm or more and 950 nm or less.

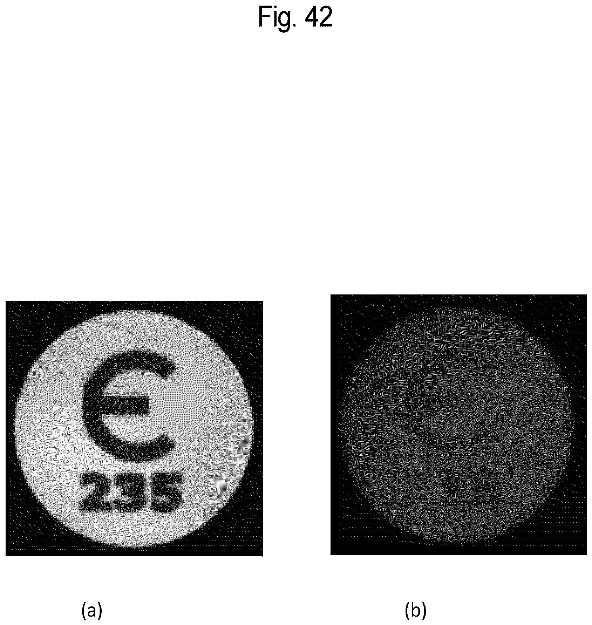

Fig. 42 is photographed data on the tablet marked with the marks according to Example 11. Fig. 42(a) is photographed data on the tablet captured by a digital camera, and Fig. 42(b) is photographed data on the tablet captured by a near infrared camera when the tablet was irradiated with near infrared light having a wavelength within a wavelength band range of 750 nm or more and 950 nm or less.

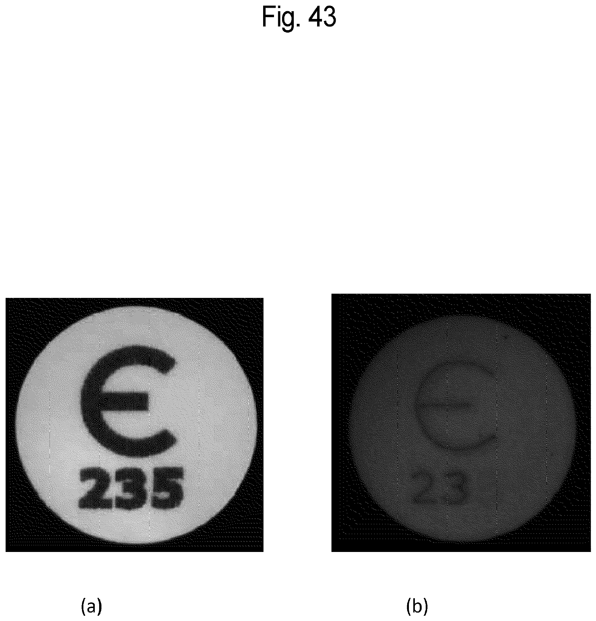

Fig. 43 is photographed data on the tablet marked with the marks according to Example 11. Fig. 43(a) is photographed data on the tablet captured by a digital camera, and Fig. 43(b) is photographed data on the tablet captured by a near infrared camera when the tablet was irradiated with near infrared light having a wavelength within a wavelength band range of 750 nm or more and 950 nm or less.

### Description of Embodiments

Hereinafter, embodiments of the present invention will be specifically described. However, it should not be construed that the following embodiments are intended to limit the present invention. Various alternative embodiments, modifications, and operation technology will be apparent to those skilled in the art from the disclosure of the present application. It should be understood that the scope of the present invention covers those various other embodiments, etc., not disclosed herein.

**A** method for determining the genuineness or spuriousness of a pharmaceutical composition for oral administration includes irradiating infrared light to a pharmaceutical composition for oral administration marked with a mark which includes an edible substance which is visually recognizable under white light irradiation as well as under infrared irradiation and a mark including an edible substance which is visually recognizable under white light irradiation and is not recognizable under infrared irradiation and determining whether the visually recognizable marks recognized on the pharmaceutical composition for oral administration irradiated with infrared light and a prepared reference mark match.

Here, the term "edible" means that the substance does no critical harm to a person who ingests the substance into the body. The edible substance is preferably a pharmaceutically acceptable substance or a hygienically acceptable substance. The pharmaceutically acceptable substances are for example listed in the Japanese pharmaceutical excipients. The hygienically acceptable substances are for example listed in Japan's Specifications and Standards for Food Additives.

The white light refers to visible light having light with various wavelengths mixed, such as sunlight. Light emitted from a normal lighting system is included in the white light. Infrared light is for example near infrared light. The wavelength band range of infrared light is for example a range of 700 nm or more or 800 nm or more. The wavelength band range of infrared light is for example a range of 2500 nm or less, 1700 nm or less, or 1000 nm or less. Alternatively, the wavelength band range of infrared light includes at least a part of the range of 700 nm or more and 800 nm or less or at least a part of the range of 700 nm or more and 900 nm or less.

The marks marked on the surface of the pharmaceutical composition for oral administration may include a character, a number, a symbol, a figure, and a pattern and also include a part of a character, a number, a symbol, a figure, and a pattern.

The pharmaceutical composition for oral administration may include titanium oxide (TiO₂) in the vicinity of the surface. A mark marked on the surface of the pharmaceutical composition for oral administration including an edible substance which is visually recognizable under white light irradiation as well as under infrared irradiation is for example a mark marked by laser irradiation on the pharmaceutical composition for oral administration using a laser irradiating device. The mark includes titanium oxide having been irradiated with laser light as an edible substance which is visually recognizable under infrared irradiation. The mark marked by the laser irradiation can be visually recognized under irradiation of infrared light having a wavelength in a wavelength band range of 700 nm or more, infrared light having a wavelength in a wavelength band range of 800 nm or more, and infrared light having a wavelength in a wavelength band range of 900 nm or more.

The laser light for marking the marks is not particularly limited and may be laser oscillated while the laser medium is in any of a solid state, a liquid state, and a gaseous state. Examples of the solid laser include a YLF laser, a YAG laser, a YVO₄ laser, and a fiber laser. Although not limited, any of fundamental, second, third, fourth, and fifth harmonic YVO₄ lasers which oscillate UV laser light is preferably used among the examples.

Here, when a mark is visually recognizable, it means that the mark is visible to an ordinary person or a photographing device.

A mark including an edible substance which is marked on a surface of a pharmaceutical composition for oral administration and is visually recognizable under white light irradiation as well as under infrared irradiation may be printed using an edible pigment including at least one of edible charcoal and black triiron tetraoxide (Fe₄O₃). Therefore, the mark printed using any of these pigments includes at least one of edible charcoal and black triiron tetraoxide (Fe₄O₃) as an edible substance which can be visually recognized under infrared irradiation. The mark printed using a pigment including at least one of edible charcoal and black triiron tetraoxide (Fe₄O₃) can be visually recognized in any of the cases in which the wavelength band range of the irradiated infrared light is a range of 700 nm or more, 800 nm or more, and 900 nm or more.

The pigment refers to white or colored powder insoluble in water, oil, etc. The pigment ink refers to ink dispersed in a solvent such as water and oil. In the pigment ink, the pigment is usually not completely dissolved in a solvent.

The pigment may be one pigment or a mixture of an any number of a plurality of pigments. In a pigment mixture, any mixing ratio of the pigments is acceptable.

The mark marked on the surface of a pharmaceutical composition for oral administration and including an edible substance which is visually recognizable under white light irradiation as well as under infrared irradiation may be a mark printed using an edible pigment including red iron oxide (Fe₂O₃). The mark printed using the pigment includes red iron oxide (Fe₂O₃) as an edible substance which is visually recognizable under infrared irradiation. The mark printed using the pigment including red iron oxide (Fe₂O₃) is visually recognizable when irradiated with infrared light having a wavelength within a wavelength band range including at least part of the range of 700 nm or more and 900 nm or less.

The mark marked on the surface of the pharmaceutical composition for oral administration and including an edible substance which is visually recognizable under white light irradiation as well as under infrared irradiation may be a mark laser-printed using an edible pigment including yellow iron sesquioxide (Fe₂O₃·H₂O). The mark laser-printed using the pigment includes the yellow iron sesquioxide (Fe₂O₃·H₂O) having been irradiated with laser light as an edible substance which is visually recognizable under infrared irradiation. The mark laser printed using the pigment including the yellow iron sesquioxide (Fe₂O₃·H₂O) is visually recognizable when irradiated with infrared light in a wavelength band range including at least a part of the range of 700 nm or more and 900 nm or less or infrared light having a wavelength within a wavelength band range including at least a part of the range of 700 nm or more and 800 nm or less. Note that the laser-printed yellow iron sesquioxide (Fe₂O₃·H₂O) is visually recognizable when irradiated with infrared light. However, the yellow iron sesquioxide (Fe₂O₃·H₂O) printed without using a laser is not visually recognizable under infrared irradiation.

The mark marked on the surface of the pharmaceutical composition for oral administration and including an edible substance which is visually recognizable under white light irradiation as well as infrared irradiation may be a mark printed using an edible pigment including sodium copper chlorophyllin. The mark printed using the pigment includes sodium copper chlorophyllin as an edible substance which is visually recognizable under infrared irradiation. The mark printed using the pigment including sodium copper chlorophyllin is visually recognizable when irradiated with infrared light in a wavelength band range including at least a part of the range of 700 nm or more and 900 nm or less or infrared light in a wavelength band range including at least a part of the range of 700 nm or more and 800 nm or less.

The mark marked on the surface of the pharmaceutical composition for oral administration and including an edible substance which is visually recognizable under white light irradiation as well as under infrared irradiation may be a mark printed using an edible pigment including indigocarmine. The mark printed using the pigment includes indigocarmine as an edible substance which is visually recognizable under infrared irradiation. The mark printed using the pigment including indigocarmine is visually recognizable when irradiated with infrared light in a wavelength band range including at least part of the range of 700 nm or more and 900 nm or less or infrared light in a wavelength range including at least a part of the range of 700 nm or more and 800 nm or less.

The mark marked on the surface of the pharmaceutical composition for oral administration and including an edible substance which is visually recognizable under white light irradiation as well as under infrared irradiation may be a mark printed using an edible pigment including black titanium oxide. The mark printed using the pigment includes black titanium oxide as an edible substance which is visually recognizable under infrared irradiation. The mark printed using the pigment including black titanium oxide is visually recognizable when irradiated with infrared light in a wavelength band range including at least a part of the range of 700 nm or more and 900 nm or less or infrared light in a wavelength band range including at least a part of the range of 700 nm or more and 800 nm or less. Note that the black titanium oxide is for example low-order titanium oxide or titanium oxynitride obtained by reducing titanium oxide. Alternatively, the black titanium oxide may be black powder obtained by mixing titanium oxynitride powder with oxynitride powder of a part of a 5A group element, a 6A group element, or a 7A group element.

When infrared light in a wavelength band range including at least part of the range of 700 nm or more and 900 nm or less is used, the surface of the pharmaceutical composition for oral administration may be marked with any combination of marks marked by laser irradiation, marks printed using pigments including at least one of edible charcoal and black triiron tetraoxide (Fe₄O₃), marks printed using pigments including red iron oxide (Fe₂O₃), marks printed using pigments including yellow iron sesquioxide (Fe₂O₃·H₂O), marks laser-printed using pigments including yellow iron sesquioxide (Fe₂O₃·H₂O), marks printed using pigments including sodium copper chlorophyllin, and marks printed using pigments including indigocarmine as marks printed using pigments including edible substances which are visually recognizable under infrared irradiation.

When infrared light having a wavelength within a wavelength band range of 900 nm or more is used, the surface of the pharmaceutical composition for oral administration may be marked with any combination of marks marked by laser irradiation, marks printed using pigments including at least one of edible charcoal and black triiron tetraoxide (Fe₄O₃) as marks including edible substances which are visually recognizable under infrared irradiation.

The mark marked on the surface of the pharmaceutical composition for oral administration and including an edible substance which is visually recognizable under white light irradiation and is not visually recognizable under infrared irradiation is a mark printed using an edible dye. The mark printed using the dye includes the dye as an edible substance which is not visually recognizable under infrared irradiation. The mark printed using the dye is not visually recognizable in any of the cases of irradiation with infrared light having a wavelength within a wavelength band range of 700 nm or more, 800 nm or more, and 900 nm or more.

Here, when the mark is not visually recognizable, it means that the mark is invisible to an ordinary person or a photographing device. The dye generally refers to an organic pigment which dyes fabric. A dye ink is an ink obtained by dissolving a dye in a solvent such as water and oil. Note however that an amount of the dye may remain undissolved in the dye ink.

The dye may include a tar dye. Historically speaking, the tar dye has been a dye including coal tar as a main constituent, while it is well known that a synthetic dye, the main constituent of which is not coal tar, is also called a "tar dye" today.

As the tar dye, at least one of erythrosine (red No. 3), New Coccine (red No. 102), tartrazine (yellow No. 4), brilliant blue FCF (blue No. 1), and sunset yellow FCF (yellow No. 5) may be used.

One dye may be used, or a mixture of any multiple dyes may be used. In the dye mixture, any mixing ratio of the dyes is acceptable.

The mark marked on the surface of the pharmaceutical composition for oral administration and including an edible substance which is visually recognizable under white light irradiation and is not visually recognizable under infrared irradiation may be a mark printed using a pigment including a tar dye aluminum lake. The mark printed using the pigment includes the tar dye aluminum lake as an edible substance which is not visually recognizable under infrared irradiation. The mark printed using the pigment including tar dye aluminum lake is not visually recognizable when the wavelength band range of the irradiated infrared light is a range of 700 nm or more, 800 nm or more, and 900 nm or more.

As a tar dye aluminum lake, at least one of erythrosine (red No. 3) aluminum lake, New Coccine (red No. 102) aluminum lake, tartrazine (yellow No. 4) aluminum lake, brilliant blue FCF (blue No. 1) aluminum lake, and sunset yellow FCF (yellow No. 5) aluminum lake can be used.

As the pigment including a tar dye aluminum lake, a pigment including one tar dye or a mixture of pigments including any number of tar dye aluminum lakes may be used. In the pigment mixture, any mixing ratio of the pigments including tar dye aluminum lakes is acceptable.

The mark marked on the surface of the pharmaceutical composition for oral administration and including an edible substance which is visually recognizable under white light irradiation and is not visually recognizable under infrared irradiation may be a mark printed using a pigment including red iron oxide. The mark printed using the pigment includes the red iron oxide an edible substance which is not visually recognizable under infrared irradiation. The mark printed using the pigment including red iron oxide can be visually recognized when irradiated with infrared light having a wavelength of a wavelength band range including at least a part of the range of 700 nm or more and 900 nm or less as described above but cannot be visually recognized when irradiated with infrared light having a wavelength of 900 nm or more.

When infrared light having a wavelength within a wavelength band range of 900 nm or more is used, any combination of marks printed using dyes and marks printed using pigments including red iron oxide may be marked on the surface of the pharmaceutical composition for oral administration as marks including edible substances which are not visually recognizable under infrared irradiation.

When printing is carried out using a pigment or a dye on the surface of the pharmaceutical composition for oral administration, an offset printer or an inkjet printer may be used, while any of other kinds of printers may be used.

A mark including an edible substance which is visually recognizable under infrared irradiation and a mark including an edible substance which is not visually recognizable under infrared irradiation can overlap each other on the surface of the pharmaceutical composition for oral administration. Alternatively, a mark including an edible substance which is visually recognizable under infrared irradiation and a mark including an edible substance which is not visually recognizable under infrared irradiation can be integrated to form one mark on the surface of the pharmaceutical composition for oral administration. For example, a certain dot or dots in one mark may be formed using an edible substance which is visually recognizable under infrared irradiation and another dot or other dots may be formed using an edible substance which is not visually recognizable under infrared irradiation. For example, when a mark represents the letter O, the right half of the letter O may be formed using an edible substance which is visually recognizable under infrared irradiation and the left half of the letter O may be formed using an edible substance which is not visually recognizable under infrared irradiation.

At least one of a pharmaceutical composition for oral administration and a packaging container for a pharmaceutical composition for oral administration may be marked with an identification number or identification code for identifying the pharmaceutical composition for oral administration.

The identification number or the identification code is a mark stamped or marked on at least one of the pharmaceutical composition for oral administration or the packaging container for a pharmaceutical composition for oral administration for the purpose of identification. The identification number or the identification code may allow the manufacturing company of the pharmaceutical composition for oral administration to be identified. Examples of the identification number or the identification code used to identify the manufacturing company may include an emblem, an abbreviated name, a sign, alphabets, Japanese characters, Chinese characters, and a mark representing the manufacturing company. The identification number or the identification code may be used to identify the product of a pharmaceutical composition for oral administration. Examples of the identification number or the identification code for identifying the product include numbers and signs.

An identification number or an identification code for a pharmaceutical composition for oral administration may correspond to the manufacturing lot or the lot number of the pharmaceutical composition for oral administration. When the identification number or the identification code is marked on the pharmaceutical composition for oral administration, the identification number or the identification code may be marked by printing using any edible ink or by laser irradiation.

The combination of a mark marked on the pharmaceutical composition for oral administration and including an edible substance which is visually recognizable under infrared irradiation and a mark including an edible substance which is not visually recognizable under infrared irradiation may be changed depending on the identification number or the identification code of the pharmaceutical composition for oral administration. Alternatively, the combination of a mark marked on a pharmaceutical composition for oral administration and including an edible substance which is visually recognizable under infrared irradiation and a mark including an edible substance which is not visually recognizable under infrared irradiation may be changed depending on the manufacturing lot or the lot number of the pharmaceutical composition for oral administration.

A true manufacturer prepares, as a reference mark, a mark which corresponds to the identification number or the identification code of a pharmaceutical composition for oral administration and is visually recognizable under infrared irradiation. Alternatively, the true manufacturer prepares, as a reference mark, a mark which corresponds to the manufacturing lot or the lot number of the pharmaceutical composition for oral administration and is visually recognizable under infrared irradiation. When a mark visually recognized under infrared irradiation on the pharmaceutical composition for oral administration match the reference mark, it is determined that the pharmaceutical composition for oral administration is authentic. When the mark visually recognized under infrared irradiation on the pharmaceutical composition for oral administration does not match the reference mark, it can be determined that the pharmaceutical composition for oral administration is counterfeit.

Only the true manufacturer is authorized to know the reference mark corresponding to a particular identification number or an identification code. A lot of combinations of an identification number or an identification code and a reference mark are available. Alternatively, only a true manufacturer is authorized to know a reference mark corresponding to a particular manufacturing lot or a lot number. A lot of combinations of a manufacturing lot or a lot number and a reference mark are available. Therefore, the mark which is visually recognizable under infrared irradiation can be used as a code, which can prevent a third party from counterfeiting the pharmaceutical composition for oral administration.

The number in the rectangle designated by 1 in the pharmaceutical composition for oral administration shown in Fig. 1(a) is marked by laser irradiation using a dye ink including New Coccine (red No. 102), and the letter of the alphabet outside the rectangle designated by 1 is marked by laser irradiation. When the pharmaceutical composition for oral administration shown in Fig. 1(a) is irradiated with infrared light having a wavelength in the range of 700 nm or more and 1700 nm or less, the number inside the rectangle designated by 1 cannot be visually recognized while the letter of the alphabet outside the rectangle designated by 1 can be visually recognized as shown in Fig. 1(b).

The numbers in the rectangle designated by 2 in the pharmaceutical composition for oral administration shown in Fig. 2(a) are marked with a pigment ink including red iron oxide, and the letter outside the rectangle designated by 2 is marked by laser irradiation. When the pharmaceutical composition for oral administration shown in Fig. 2(a) is irradiated with infrared light having a wavelength in the range of 900 nm or more and 1700 nm or less, the numbers in the rectangle designated by 2 cannot be visually recognized while the letter outside the rectangle designated by 2 can be visually recognized as shown in Fig. 2(b).

The numbers in the rectangle designated by 3 are marked using a pigment ink including edible charcoal on the pharmaceutical composition for oral administration shown in Fig. 3(a), and the letter of the alphabet outside the rectangle designated by 3 are marked by laser irradiation. When the pharmaceutical composition for oral administration shown in Fig. 3(a) is irradiated with infrared light having a wavelength in the range of 700 nm or more and 1700 nm or less, the numbers in the rectangle designated by 3 and the letter outside the rectangle designated by 3 can be visually recognized as shown in Fig. 3(b).

The letter E is finely marked by laser irradiation on a pharmaceutical composition for oral administration as shown in Fig. 4(a), and the letter E is marked thereon thickly with a dye ink including New Coccine (red No. 102). When the pharmaceutical composition for oral administration shown in Fig. 4(a) is irradiated with infrared light having a wavelength in the range of 700 nm or more and 1700 nm or less, the thick mark marked with the dye ink including New Coccine (red No. 102) cannot be visually recognized and the thin mark marked by laser irradiation can be visually recognized as shown in Fig. 4(b).

In the pharmaceutical compositions for oral administration shown in Figs. 5(a) and 6(a), the numbers in the rectangles designated by 4 are marked by the dye ink including New Coccine (red No. 102), and the numbers outside the rectangles designated by 4 are marked using a pigment ink including at least one of edible charcoal and black triiron tetraoxide (Fe₄O₃). When the pharmaceutical compositions for oral administration shown in Figs. 5(a) and 6(a) are irradiated with infrared light having a wavelength in the range of 700 nm or more and 1700 nm or less, the numbers in the rectangles designated by 4 cannot be visually recognized while the numbers outside the rectangles designated by 4 can be visually recognized as shown in Figs. 5(b) and 6(b).

In the pharmaceutical composition for oral administration shown in Fig. 7(a), the part of the letter E in the rectangle designated by 5 is marked using a dye ink including New Coccine (red No. 102), and the part outside the rectangle designated by 5 is marked using a pigment ink including at least one of edible charcoal and black triiron tetraoxide (Fe₄O₃). When the pharmaceutical composition for oral administration shown in Fig. 7(a) is irradiated with infrared light having a wavelength in the range of 700 nm or more and 1700 nm or less, the part of the mark E in the rectangle designated by 5 cannot be visually recognized while the part outside the rectangle designated by 5 can be visually recognized as shown in Fig. 7(b).

In the pharmaceutical composition for oral administration shown in Fig. 8(a), the part of the mark E in the rectangle designated by 6 is marked using a dye ink including New Coccine (red No. 102), the part outside the rectangle designated by 6 is marked using a pigment ink including red iron oxide. When the pharmaceutical composition for oral administration shown in Fig. 8(a) is irradiated with infrared light having a wavelength in the range of 700 nm or more and 900 nm or less, the part of the mark E in the rectangle designated by 6 cannot be visually recognized while the part outside the rectangle designated by 6 can be visually recognized as shown in Fig. 8(b).

In the pharmaceutical composition for oral administration shown in Fig. 9(a), the numbers in the rectangle designated by 7 are marked using a dye ink including New Coccine (red No. 102), the numbers outside the rectangle designated by 7 are marked using a pigment ink including red iron oxide. When the pharmaceutical composition for oral administration shown in Fig. 9(a) is irradiated with infrared light having a wavelength in the range of 700 nm or more and 900 nm or less, the numbers in the rectangle designated by 7 cannot be visually recognized while the part outside the rectangle designated by 7 can be visually recognized as shown in Fig. 9(b).

In the pharmaceutical composition for oral administration shown in Fig. 10(a), the part of the mark E in the rectangle designated by 8 is marked using a mixture ink including a dye ink including New Coccine (red No. 102) and a pigment ink including red iron oxide, and the part other than the rectangle designated by 8 is marked by laser irradiation. When the pharmaceutical composition for oral administration shown in Fig. 10(a) is irradiated with infrared light having a wavelength in the range of 700 nm or more and 900 nm or less, the part of the mark E in the rectangle designated by 8 cannot be visually recognized while the part outside the rectangle designated by 8 can be visually recognized as shown in Fig. 10(b).

In the pharmaceutical composition for oral administration shown in Fig. 11(a), the numbers in the rectangles designated by 8 are marked using a mixture ink including a dye ink including New Coccine (red No. 102) and a pigment ink including red iron oxide, and the numbers outside the rectangle designated by 8 are marked by laser irradiation. When the pharmaceutical composition for oral administration shown in Fig. 11(a) is irradiated with infrared light having a wavelength in the range of 700 nm or more and 900 nm or less, the numbers in the rectangles designated by 8 is visually recognizable but the numbers look thinner while the part outside the rectangles designated by 8 can be visually recognized and unchanged as shown in Fig. 11(b).

In the pharmaceutical composition for oral administration shown in Fig. 12(a), the letter E is marked finely using a pigment ink including black triiron tetraoxide (Fe₄O₃) and then the letter E is thickly marked thereon using a black dye ink. When the pharmaceutical composition for oral administration shown in Fig. 12(a) is irradiated with infrared light having a wavelength in the range of 700 nm or more and 1700 nm or less, the thick mark marked using the black dye ink cannot be visually recognized while the thin letter E marked using the pigment ink including the black triiron tetraoxide (Fe₄O₃) can be visually recognized as shown in Fig. 12(b).

In the pharmaceutical composition for oral administration shown in Fig. 13(a), the numbers are marked thinly using a pigment ink including black triiron tetraoxide (Fe₄O₃) and then the numbers thickly marked thereon using a black dye ink. When the pharmaceutical composition for oral administration shown in Fig. 13(a) is irradiated with infrared light having a wavelength in the range of 700 nm or more and 1700 nm or less, the thick mark marked using the black dye ink cannot be visually recognized while the thin mark marked using the pigment ink including the black triiron tetraoxide (Fe₄O₃) can be visually recognized as shown in Fig. 13(b).

In the pharmaceutical composition for oral administration shown in Fig. 14(a), the letter E includes an alternate arrangement of an intermittently marked part using a pigment ink including black triiron tetraoxide (Fe₄O₃) and an intermittently marked part using a black dye ink. When the pharmaceutical composition for oral administration shown in Fig. 14(a) is irradiated with infrared light having a wavelength in the range of 700 nm or more and 1700 nm or less, the intermittently marked part using the black dye ink cannot be visually recognized while the intermittently marked part using the pigment ink including the black triiron tetraoxide (Fe₄O₃) can be visually recognized as shown in Fig. 14(b).

In the pharmaceutical composition for oral administration shown in Fig. 15(a), the numbers include an alternate arrangement of an intermittently marked part using a pigment ink including black triiron tetraoxide (Fe₄O₃) and then an intermittently marked part using a black dye ink. When the pharmaceutical composition for oral administration shown in Fig. 15(a) is irradiated with infrared light having a wavelength in the range of 700 nm or more and 1700 nm or less, the intermittently marked part using the black dye ink cannot be visually recognized while the intermittently marked part using the pigment ink including the black triiron tetraoxide (Fe₄O₃) can be visually recognized as shown in Fig. 15(b).

Fig. 16 schematically illustrates an example of procedure of a method for determining the genuineness or spuriousness of a pharmaceutical composition for oral administration according to the embodiment. In an genuine pharmaceutical composition for oral administration with a lot number "12345" shown in Fig. 16(a), the mark "E" includes an edible substance which can be visually recognized under infrared irradiation. The mark "4" in the identification number "243" corresponding to the lot number "12345" includes an edible substance which can be visually recognized under infrared radiation, the marks "2" and "3" include an edible substance which cannot be visually recognized under infrared irradiation.

Therefore, when the genuine pharmaceutical composition for oral administration shown in Fig. 16(a) is irradiated with infrared light, the mark "E" and the mark "4" are visually recognized while the marks "2" and "3" are not visually recognized as shown in Fig. 16(b). When this is compared to the reference marks of the pharmaceutical composition for oral administration with the lot number "12345" shown in Fig. 16(c), the visually recognized marks match the reference marks, and the pharmaceutical composition for oral administration shown in Fig. 16(a) can be authenticated.

Meanwhile, assume that a counterfeit of the pharmaceutical composition for oral administration with a lot number "12345" shown in Fig. 17(a) includes marks, all of which is visually recognizable under infrared irradiation. In this case, if the counterfeit pharmaceutical composition for oral administration shown in Fig. 17(a) is irradiated with infrared light, the marks "2" and "3" are both visually recognized. When this is compared to the reference marks of the pharmaceutical composition for oral administration with the lot number "12345" shown in Fig. 17(c), the visually recognized marks do not match the reference marks, and therefore the pharmaceutical composition for oral administration shown in Fig. 17(a) can be determined as a counterfeit.

The pharmaceutical composition for oral administration needs only be in a prescribed form which enables marking, except a powder state which makes marking difficult, and the form is not particularly limited. Examples of the form of the pharmaceutical composition for oral administration include, but not limited to, a tablet such as an uncoated tablet, an oral fast disintegrating tablet, or a dry-coated tablet, as well as a pill, a troche, a chewable tablet, and a capsule. The form is more preferably a tablet or a capsule.

The pharmaceutical composition for oral administration may be produced in the form of tablets by molding a mixture including a drug, a flavor, an additive, and a colorant and marking a mark on a surface thereof.

The pharmaceutical composition for oral administration in the form of tablets is manufactured by producing a mixture including a drug, a flavor, an additive, and a colorant using a V-type blender (for example, manufactured by Tokuju Corporation, Fuji Paudal Co., Ltd., or Dalton Corporation), a tumbler blender (for example manufactured by Dalton Corporation or Daiko Seiki Co., Ltd.) or a high-speed stirring mixer (for example manufactured by Okada Seiko Co., Ltd., Nara Machinery Co., Ltd., or Powrex Corporation) and molding the resulting powder or granulate mixture. Examples of molds include compression molding and mold-molding. A tableting machine such as a single-punch tableting machine, a rotary type tableting machine, and a dry-coated tablet tableting machine may be used for compression molding. A mold-molding machine is used for mold-molding. In the mold-molding, a product is manufactured by filling a mold with a suspension or slurry dispersed in a solvent such as water and drying the solvent.

The pharmaceutical composition for oral administration may include a coating layer. Examples of the coating layer include a film-coating layer and a sugar-coating layer. The marks may be marked on the coating layer of the pharmaceutical composition for oral administration. Alternatively, a coating layer may be provided on the mark in order to protect the mark in the pharmaceutical composition for oral administration. In this case, the coating layer is preferably transparent. Examples of the pharmaceutical composition for oral administration having a coating layer include a film-coated tablet, a sugar-coated tablet, a dry-coated tablet, and a pill. The film-coating layer and the sugar-coating layer can be produced, but not limited to, for example by a coating granulation method, a film coating method, a sugar coating method, and a compression coating method.

The pharmaceutical composition for oral administration may be provided as a capsule. For example, the pharmaceutical composition for oral administration as a capsule is manufactured by filling a content containing a drug, edible fat and oil, a flavor, and an additive in a capsule coating layer and marking a mark on the surface of the capsule coating layer. The capsule may be a soft capsule or a hard capsule. The content may be in the form of powder, granules, liquid, and slurry. Examples of the capsule coating layer include, but not limited to, a gelatin capsule, an agar capsule, a hypromellose (HPMC) capsule, a pullulan capsule, and a starch capsule. When the capsule is filled with the content, for example a rotary die type capsule filling machine, a seamless capsule filling machine, and a hard capsule filling machine (Qualicaps Co., Ltd.) may be used.

The marked pharmaceutical composition for oral administration is subjected to inspection for example using a character inspection machine and a metal inspection machine and then discharged onto a transport drum or filled in a packaging container. The timing for marking the mark on the pharmaceutical composition for oral administration is not particularly limited and the marking can be freely incorporated into the process of producing the pharmaceutical composition for oral administration.

For example, the mark can be marked on the pharmaceutical composition for oral administration in the process of moving on the transfer line before being filled into the package, and therefore the process may also be applied to so-called continuous production. Alternatively, the pharmaceutical composition for oral administration is automatically spread in a flat pan for storing a fixed amount of the pharmaceutical composition for oral administration in the transport line, and then the transport line is stopped, so that the pharmaceutical composition for oral administration may be marked with a mark.

Alternatively, the mark may be marked on the pharmaceutical composition for oral administration by batch processing using an automatic marking device having a laser oscillator. More specifically, the pharmaceutical composition for oral administration loaded in the hopper of the marking device is sequentially supplied from the hopper to the inspection unit, and then, the pharmaceutical composition for oral administration is checked for stains, chips, cracks, etc., on the front and back surfaces thereof using a video inspection machine in the inspection unit, then one or more pieces of oral administration pharmaceutical compositions are moved to a location for marking, and the oral administration pharmaceutical composition can be marked on the surface.

The step of marking a mark on the pharmaceutical composition for oral administration may be carried out multiple times depending on the number of colors. Alternatively, marking may be performed once using a laser transfer film provided with a plurality of different transfer layers corresponding to multiple colors for marking.

The method for determining the genuineness or spuriousness of a pharmaceutical composition for oral administration according to the embodiment may be performed by a system for determining the genuineness or spuriousness of a pharmaceutical composition for oral administration.
The system for determining the genuineness or spuriousness of a pharmaceutical composition for oral administration includes an irradiator configured to irradiate infrared light to a pharmaceutical composition for oral administration marked with a mark including an edible substance which is visually recognizable under white light irradiation as well as under infrared irradiation and a mark including an edible substance which is visually recognizable under white light irradiation and is not visually recognizable under infrared irradiation and a determining unit configured to determine whether the visually recognizable marks recognized on the pharmaceutical composition for oral administration irradiated with infrared light and a prepared reference mark match.

The determining unit may be implemented by a central processing unit (CPU). The system for determining the genuineness or spuriousness of a pharmaceutical composition for oral administration may further include a photographing device. The photographing device takes a photo of the pharmaceutical composition for oral administration irradiated with the infrared light. The photographing device transmits a captured image to the determining unit. The determining unit determines whether the mark visually recognized in the image received from the photographing device and the prepared reference mark match. The system for determining the genuineness or spuriousness of a pharmaceutical composition for oral administration may further include an output unit which outputs a determination result by the determining unit and a storing unit which stores the determining result by the determining unit.

### Example

Hereinafter, the embodiment of the present invention will be described more in detail with reference to examples.

Note however that the embodiment of the present invention is not limited to the following examples in any way.

### [Example 1]

As a tablet, a 10-mg tablet of commercially available Pariet (registered trademark) was prepared. The Pariet tablet is an enteric coated preparation. The surface of the Pariet tablet is covered with a film containing hypromellose phthalate, titanium oxide (TiO₂), and yellow iron sesquioxide. The tablet was irradiated with UV laser light and marked with the marks. The laser irradiation device was an irradiating device manufactured by Photonics Industries International, Inc. The laser light was irradiated under the following condition.
Laser: YVO₄ laser
Type: DS20H-355
Wavelength: 355 nm
Peak power: 6 kW
Average power at 20 kHz: 3 W
Pulse width at 20 kHz: 25 ns
Pulse energy at 20 kHz: 0.15 mJ
Beam mode: TEM₀₀-M² < 1.1
Polarization extinction ratio (Polarization ratio): 100:1 Horizontal
Beam size: 0.9 mm
Beam spread angle (full angle): 1.3 mrad
Inter-pulse stability: 3% rms
Long-term instability: +/- 3%
Pointing stability: < 50 µrad
Pulse repetition rate: single shot to 100 kHz

Figs. 18 and 19 show Photographed data on the marked tablet according to Example 1. Fig. 18(a) illustrates the photographed data on the tablet according to Example 1 photographed by a digital camera capable of photographing a normal visible light image under sunlight. Fig. 18(b) is photographed data of a tablet according to Example 1 photographed by a near infrared camera under sunlight, without irradiating the tablet with near infrared light, and the mark marked by UV laser light was visually recognized.

Fig. 19 is photographed data on the tablet according to Example 1 photographed using a near infrared camera when near infrared light with a wavelength within a wavelength band range of 800 nm or more and 1000 nm or less was irradiated to the tablet in a dark room. Under near infrared irradiation, the mark marked by UV laser light was visually recognized.

### [Example 2]

A tablet was marked with a mark in the same condition as Example 1 except that the used tablet was an uncoated tablet including 58.5% lactose, 30% crystalline cellulose, 10% corn starch, 1% titanium oxide (TiO₂), and 0.5% magnesium stearate instead of a 10-mg tablet of Pariet.

Photographed data on the marked tablet according to Example 2 is shown in Fig. 20 and Fig. 21. Fig. 20(a) is photographed data on a tablet according to Example 2 captured by a digital camera capable of capturing a normal visible light image under sunlight. Fig. 20 (b) is photographed data on a tablet according to Example 2 captured by a near infrared camera under sunlight without irradiating the tablet with near infrared light. Under sunlight, the mark marked by UV laser light was visually recognized.

Fig. 21 is photographed data on a tablet according to Example 2 photographed by a near infrared camera when the tablet was irradiated with near infrared light having a wavelength within a wavelength band range of 800 nm or more and 1000 nm or less in a dark room. When irradiated with near infrared light, the mark marked by UV laser light was visually recognized.

### [Example 3]

As a tablet, a 5-mg orally disintegrating tablet (D tablet) of commercially available Aricept (registered trademark) was prepared. The surface of the 5 mg Aricept D tablet was printed with E248 in small letters and 5 in large letter using a pigment ink including edible charcoal as a main constituent.

Then, a laser transfer film was prepared. The film included a base material made of a low density polyethylene film and a transfer layer as a colored film including ethyl cellulose (40 parts by mass), red No. 3 (40 parts by mass), and triethyl citrate (20 parts by mass) provided on the base material. The laser transfer film was irradiated with laser light on the base material side and the transfer layer is transferred onto the surface of the tablet, so that the surface of the tablet was marked with the mark
" 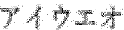 " and the mark "A8". A laser irradiation device manufactured by Photonics Industries International, Inc., was used. The laser light was irradiated under the following condition.
Laser: YVO₄ laser
Type: DS20H-355
Wavelength: 355 nm
Peak power: 6 kW
Average power at 20 kHz: 3 W
Pulse width at 20 kHz: 25 ns
Pulse energy at 20 kHz: 0.15 mJ
Beam mode: TEM₀₀-M²< 1.1
Polarization extinction ratio (polarization ratio): 100:1 Horizontal
Beam size: 0.9 mm
Beam spread angle (full angle): 1.3 mrad
Inter-pulse stability: 3% rms
Long-term instability: +/- 3%
Pointing stability: < 50 µrad
Pulse repetition rate: single shot to 100 kHz

Figs. 22 and 23 show photographed data on the marked tablet according to Example 3. Fig. 22(a) illustrates photographed data on the tablet according to Example 3 captured by a digital camera capable of photographing a normal visible light image under sunlight. Fig. 22(b) is photographed data on the tablet according to Example 3 captured by a near infrared camera under sunlight without irradiating the tablet with near infrared light. Under sunlight, the mark marked using edible charcoal and the mark marked using red No. 3 were visually recognized.

Fig. 23 shows photographed data on the tablet according to Example 3 captured by a near infrared camera when the tablet was irradiated with near infrared light having a wavelength within a wavelength band range of 800 nm or more and 1000 nm or less in a dark room. Under near infrared irradiation, the mark marked using edible charcoal was visually recognized. However, the mark marked using red No. 3 could not be visually recognized under near infrared irradiation.

### [Example 4]

The tablet was marked under the same irradiation condition as Example 3 except that a 10-mg tablet of Aricept D was used as the tablet instead of a 5-mg tablet of Aricept D.

Figs. 24 and 25 show photographed data on the marked tablet according to Example 4. Fig. 24(a) shows photographed data on the tablet according to Example 4 obtained by a digital camera capable of photographing a normal visible light image under sunlight. Fig. 24(b) shows photographed data on the tablet according to Example 4 captured by a near infrared camera under sunlight without irradiating the tablet with near infrared light. Under sunlight, the mark marked using edible charcoal and the mark marked using red No. 3 were visually recognized.

Fig. 25 shows photographed data on the tablet according to Example 4 captured by a near infrared camera when the tablet was irradiated with near infrared light having a wavelength within a wavelength band range of 800 nm or more and 1000 nm or less in a dark room. Under near infrared irradiation, the mark marked using edible charcoal was visually recognized. However, the mark marked using red No. 3 could not be visually recognized under near infrared irradiation.

### [Example 5]

A film-coated tablet was prepared as a tablet. The uncoated tablet before being film-coated was manufactured by mixing 0.5% magnesium stearate into a mixture of lactose and crystalline cellulose and tableting the mixture into a tablet having a weight of 125 mg. The film coating layer contained hydroxypropyl cellulose as a base material and 1.5% of macrogol 6000 and 1.5% of titanium oxide (TiO₂).

The tablet was marked with the mark shown in Fig. 26 on the surface using a red dye ink, a red pigment ink, and an ink jet printer manufactured by Qualicaps Co., Ltd. The red dye ink contained red No. 102, yellow No. 4, and blue No. 1 as pigment components. The red pigment ink contained red iron oxide as a pigment component. In Fig. 26, the marks enclosed in rectangles designated by 101 and 102 were marked using the red dye ink. The marks not enclosed in the rectangles designated by 101 and 102 were marked using the red pigment ink.

Figs. 27 and 28 show photographed data on the marked tablet according to Example 5. Fig. 27(a) shows photographed data on the tablet according to Example 5 captured by a digital camera capable of photographing a normal visible light image under sunlight. Fig. 27(b) shows photographed data on the tablet according to Example 5 captured by a near infrared camera under sunlight without irradiating the tablet with near infrared light. Under sunlight, the mark marked with the red dye ink and the mark marked with the red pigment ink were visually recognized.

Fig. 28(a) shows photographed data on the tablet according to Example 5 captured by a near infrared camera when the tablet was irradiated with near infrared light having a wavelength within a wavelength band range of 750 nm or more and 900 nm or less in a dark room. When near infrared light having a wavelength within a wavelength band range of 750 nm or more and 900 nm or less was irradiated, the mark marked using the red pigment ink was visually recognized. However, when near infrared light having a wavelength within a wavelength band range of 750 nm or more and 900 nm or less was irradiated, the mark marked using the red dye ink could not be visually recognized.

Fig. 28(b) shows photographed data on the tablet according to Example 5 by a near infrared camera when the tablet was irradiated with near infrared light having a wavelength within a wavelength band range of 900 nm or more and 1000 nm or less excluding less than 900 nm. When near infrared light having a wavelength within a wavelength band range of 900 nm or more and 1000 nm or less is irradiated, the mark marked using the red pigment ink and the mark marked using the red dye ink could not be visually recognized.

### [Example 6]

As a tablet, the same film-coated tablet as Example 5 was prepared. Using a black pigment ink, a red pigment ink, and an ink jet printer made by Qualicaps Co., Ltd. the mark shown in Fig. 29 was marked on the tablet surface. The black pigment ink included edible charcoal as a pigment component. The red pigment ink contained red iron oxide as a pigment component. In Fig. 29, the mark part enclosed by the rectangle designated by 103 was marked using the red pigment ink. The mark part not enclosed by the rectangle designated by 103 was marked using the black pigment ink.

Figs. 30 and 31 show photographed data on the marked tablet according to Example 6. Fig. 30(a) shows photographed data on the tablet according to Example 6 captured by a digital camera capable of photographing a normal visible light image under sunlight. Fig. 30(b) is photographed data on the tablet according to Example 6 captured by a near infrared camera under sunlight without irradiating the tablet with near infrared light. Under sunlight, the mark marked using the black pigment ink and the mark marked using the red pigment ink were visually recognized.

Fig. 31 shows photographed data on the tablet according to Example 6 captured by a near infrared camera when the tablet was irradiated with a wavelength within a wavelength band range of 900 nm or more and 1000 nm or less excluding less than 900 nm in a dark room. When near infrared light having a wavelength within a wavelength band range of 900 nm or more and 1000 nm or less was irradiated, the mark part marked using the black pigment ink was visually recognized. However, when irradiated with near infrared light having a wavelength in the range of 900 nm or more and 1000 nm or less, the mark part marked using the red pigment ink could not be visually recognized.

### [Reference Example]

As a tablet, an uncoated tablet consisting of 59.5% lactose, 30% crystalline cellulose, 10% corn starch, and 0.5% magnesium stearate was prepared. A laser transfer film is prepared, and the film included a base material of made of a low density polyethylene film and a transfer layer as a colored film including Eudragit E 100 (30 parts by mass), yellow No. 5 aluminum lake (15 parts by mass), and triethyl citrate (5 parts by mass) provided on the base material. The laser transfer film was irradiated with laser light on the base material side, so that the transfer layer was transferred onto the surface of the tablet. The mark shown in Fig. 32(a) was marked on the tablet surface. Fig. 32(b) is photographed data on the tablet according to the reference example captured by a near infrared camera when the tablet was irradiated with near infrared light having a wavelength within a wavelength band range of 750 nm or more and 950 m or less. In this case, the mark marked using the yellow No. 5 aluminum lake could not be visually recognized.

### [Example 7]

As a tablet, an uncoated tablet including 59.5% lactose, 30% crystalline cellulose, 10% corn starch, and 0.5% magnesium stearate was prepared. In addition, a laser transfer film was prepared, and the film included a base material made of a low density polyethylene film and a transfer layer provided on the base material and including 50 parts by mass of Eudragit E-100 (registered trademark), 33.3 parts by mass of a dye, and 16.7 parts by mass of triethyl citrate. In the laser transfer film, the dye was any of red No. 3 aluminum lake, yellow iron-sesquioxide, and sodium copper chlorophyllin.

The transfer layer was transferred onto the tablet surface by irradiating the laser transfer film on the base material side (low density polyethylene film side of the base material) with laser light under the following condition, and the surface of the uncoated tablet was marked with the mark
" 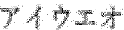 " and the mark A8. A laser beam irradiation device manufactured by Photonics Industries International, Inc., was used. The laser light was irradiated under the following condition.
Laser: YVO₄ laser
Type: DS20H-355
Wavelength: 355 nm
Peak power: 6 kW
Average power at 20 kHz: 3 W
Pulse width at 20 kHz: 25 ns
Pulse energy at 20 kHz: 0.15 mJ
Beam mode: TEM₀₀-M²< 1.1
Polarization extinction ratio (Polarization ratio): 100:1 Horizontal
Beam size: 0.9 mm
Beam spread angle (full angle): 1.3 mrad
Inter-pulse stability: 3% rms
Long-term instability: +/- 3%
Pointing stability: < 50 µrad
Pulse repetition rate: single shot to 100 kHz

Figs. 33 to 35 show photographed data on the marked tablet according to Example 7. Fig. 33(a) is photographed data on the tablet according to Example 7 captured by a digital camera capable of photographing a normal visible light image under sunlight when the pigment was a red No. 3 aluminum lake. Fig. 33(b) is photographed data on the tablet according to Example 7 captured by a near infrared camera when the pigment was red No. 3 aluminum lake and the tablet was irradiated with near infrared light having a wavelength i within a wavelength band range of 750 nm or more and 950 nm or less in a dark room. Under near infrared irradiation, the mark marked using the red No. 3 aluminum lake could not be visually recognized.

Fig. 34(a) is photographed data on a tablet according to Example 7 captured by a digital camera capable of capturing a normal visible light image under sunlight when the pigment was yellow iron sesquioxide. Fig. 34(b) is photographed data on the tablet according to Example 7 captured using a near infrared camera when the pigment was yellow iron sesquioxide and the tablet was irradiated with near infrared light having a wavelength within a wavelength band range of 750 nm or more and 950 nm or less in a dark room. Under near infrared irradiation, the mark marked using yellow iron sesquioxide was visually recognized.

Fig. 35 (a) is photographed data on a tablet according to Example 7 captured by a digital camera capable of photographing a normal visible light image under sunlight when the pigment is sodium copper chlorophyllin. Fig. 35(b) shows a tablet photographed by a near infrared camera when the tablet according to Example 7 is irradiated with near infrared light having a wavelength within a wavelength band range of 750 nm or more and 950 nm or less in a dark room when the pigment is sodium copper chlorophyllin. Under near infrared irradiation, the mark marked using sodium copper chlorophyllin was visually recognized.

### [Example 8]

An uncoated tablet containing 59.5% lactose, 30% crystalline cellulose, 10% corn starch, and 0.5% magnesium stearate was prepared. Also, a laser transfer film as a colored film was prepared and the film included a base material made of a low density polyethylene film, 42.9 parts by mass of Eudragit RS 100, 42.9 parts by mass of a pigment, and 14.3 parts by mass of triethyl citrate disposed on the base material. In the laser transfer film, the pigment was either indigocarmine or brilliant blue.

**The** laser transfer film was irradiated with laser light on the base material side (the low density polyethylene film side of the base material) under the following conditions, the transfer layer was transferred to the tablet surface, and the mark was marked on the surface of the uncoated tablet using indigocarmine as the pigment while when the pigment was brilliant blue, the mark of letters was marked. As a laser beam irradiation device manufactured by Photonics Industries International, Inc., was used. The laser light was irradiated under the following condition.
Laser: YVO₄ laser
Type: DS20H-355
Wavelength: 355 nm
Peak power: 6 kW
Average power at 20 kHz: 3 W
Pulse width at 20 kHz: 25 ns
Pulse energy at 20 kHz: 0.15 mJ
Beam mode: TEM₀₀-M²< 1.1
Polarization extinction ratio (Polarization ratio): 100:1 Horizontal
Beam size: 0.9 mm
Beam spread angle (full angle): 1.3 mrad,
Inter-pulse stability: 3% rms
Long-term instability: +/- 3%
Pointing stability: < 50 µrad
Pulse repetition rate: single shot to 100 kHz

Figs. 36 and 37 show photographed data on the marked tablet according to Example 8. Fig. 36(a) shows photographed data on the tablet according to Example 8 captured by a digital camera capable of photographing a normal visible light image under sunlight when the pigment was indigocarmine. Fig. 36(b) shows photographed data on the tablet according to Example 8 captured by a near infrared camera when the tablet was irradiated with near infrared light having a wavelength within a wavelength band range of 750 nm or more and 950 nm or less in a dark room and the pigment was indigocarmine. The mark marked using indigocarmine was visually recognized under near infrared irradiation.

Fig. 37(a) is photographed data on a tablet according to Example 8 captured by a digital camera capable of photographing a normal visible light image under sunlight when the pigment was brilliant blue. Fig. 37(b) shows photographed data on the tablet according to Example 8 captured by a near infrared camera when the tablet was irradiated with near infrared light having a wavelength within a wavelength band range of 750 nm or more and 950 nm or less in a dark room and the pigment was brilliant blue. The mark marked using the brilliant blue could not be visually recognized under near infrared irradiation.

### [Example 9]

An uncoated tablet including 59.5% lactose, 30% crystalline cellulose, 10% corn starch, and 0.5% magnesium stearate was prepared. An ink was produced by dissolving and dispersing 42.9 parts by mass of Eudragit RS 100, 42.9 parts by mass of indigocarmine, and 14.3 parts by mass of triethyl citrate in 100 parts by mass of ethanol. The uncoated tablet was stamped using the ink and dried.

Fig. 38 shows photographed data on the marked tablet according to Example 9. Fig. 38(a) is photographed data on the tablet according to Example 9 captured by a digital camera capable of photographing a normal visible light image under sunlight. Fig. 38(b) is photographed data on the tablet according to Example 9 captured by a near infrared camera when the tablet was irradiated with near infrared light having a wavelength within a wavelength band range of 750 nm or more and 950 nm or less in a dark room. The mark marked using indigocarmine was visually recognized under near infrared irradiation.

### [Example 10]

A white tablet and a colored tablet were prepared.

The white tablet was prepared by mixing 0.5% magnesium stearate with a mixture of lactose and microcrystalline cellulose and tableting the mixture into a tablet having a weight of 125 mg. The film coating layer had hydroxypropyl cellulose as a base material and contained 1.5% of macrogol 6000 and 1.5% of titanium oxide (TiO₂).

As the colored tablet, a 10-mg commercially available Fycompa (registered trademark) tablet was prepared. The surface of the Fycompa (registered trademark) tablet was coated with a film containing hypromellose, titanium oxide (TiO₂), and red iron sesquioxide.

The white tablet and the colored tablet were each irradiated with green laser light and marked with marks. A laser light irradiation device manufactured by Keyence Corporation was used. The laser light was irradiated under the following condition.
Laser: YVO₄ laser
Type: MD-S9910A
Console: MC-P1
Printing method: XYZ three-axis simultaneous scanning
Wavelength: 532 nm
Average power: 6 W
Q switch frequency: 1 kHz to 400 kHz

Figs. 39 and 40 show photographed data on the marked tablets according to Example 10. Fig. 39(a) is photographed data on the white tablet according to Example 10 captured by a digital camera capable of photographing a normal visible light image under sunlight. Fig. 39(b) is photographed data on the white tablet according to Example 10 captured by a near infrared camera when the tablet was irradiated with near infrared light having a wavelength within a wavelength band range of 750 nm or more and 950 nm or less in a dark room. The mark marked by laser irradiation was visually recognized under near infrared irradiation.

Fig. 40(a) shows photographed data on the colored tablet according to Example 10 captured by a digital camera capable of photographing a normal visible light image under sunlight. Fig. 40(b) is photographed data on the colored tablet according to Example 10 captured by a near infrared camera when the tablet was irradiated with near infrared light having a wavelength within a wavelength band range of 750 nm or more and 950 nm or less in a dark room. The mark marked by laser irradiation was visually recognized under near infrared irradiation.

### [Example 11]

A mixture obtained by mixing 0.5% magnesium stearate in a mixture of lactose and crystalline cellulose was tableted into a tablet with a weight of 125 mg. The tablet was provided with a film coating layer having hydroxypropyl cellulose as a base material and containing 1.5 % of Macrogol 6000 and 1.5% of titanium oxide (TiO₂).

The film coating layer on the tablet was printed with a mark using a UV marking device manufactured by Qualicaps Co., Ltd, and then a mark is further printed using a blue dye ink on the printed mark by an ink jet printer manufactured by Qualicaps Co., Ltd. The laser light was irradiated under the following condition.
Laser: YVO₄ laser
Type: Talon 355-15
Wavelength: 355 nm
Frequency: 30 kHz
Average power: 1.25 W
Scanner speed: 3000 mm/sec
Expander: 13.0 mm

Figs. 41 to 43 show photographed data on the marked tablet according to Example 11. Fig. 41(a) shows photographed data on the tablet according to Example 11 captured by a digital camera capable of photographing a normal visible light image under sunlight when E235 was printed with UV laser light and then E235 was printed using a blue dye ink thereon. Fig. 41(b) is photographed data on the tablet according to Example 11 captured by a near infrared camera when the tablet printed with E235 using UV laser light and printed again with E235 using the blue dye ink was irradiated with near infrared light having a wavelength i within a wavelength band range of 750 nm or more and 950 nm or less in a dark room. The mark marked using the UV laser light was visually recognized under near infrared irradiation.

Fig. 42(a) is photographed data on the tablet according to Example 11 captured by a digital camera capable of photographing a normal visible light image under sunlight when the tablet was printed with E 35 using UV laser light and then printed with E235 using the blue dye ink in the same location. Fig. 42(b) is photographed data on the tablet according to Example 11 captured by a near infrared camera when the tablet printed with E 35 using UV laser light and then E235 using blue dye ink in the same location was irradiated with near infrared light having a wavelength within a wavelength band range of 750 nm or more and 950 nm or less in a dark room. The mark marked using the UV laser light was visually recognized under near infrared irradiation. The mark marked using the blue dye ink could not be visually recognized.

Fig. 43(a) is photographed data on the tablet according to Example 11 captured by a digital camera capable of photographing a normal visible light image under sunlight when the tablet was printed with E23 using UV laser light and then with E235 using the blue dye ink in the same location. Fig. 43(b) is photographed data on the tablet according to Example 11 captured by a near infrared camera when the tablet printed with E23 using UV laser light and then with the E235 using blue dye ink in the same location was irradiated with near infrared light having a wavelength within a wavelength band range of 750 nm or more and 950 nm or less in a dark room. The mark marked using the UV laser light was visually recognized. The mark marked using the blue dye ink could not be visually recognized.

## Claims

1. A method for determining the genuineness or spuriousness of a pharmaceutical composition for oral administration, comprising
irradiating a pharmaceutical composition for oral administration with infrared light, the pharmaceutical composition for oral administration being marked with a mark including an edible substance which is visually recognizable under white light irradiation as well as under infrared irradiation and a mark including an edible substance which is visually recognizable under white light irradiation and is not visually recognizable under infrared irradiation; and
determining whether a visually recognizable mark visually recognized on the pharmaceutical composition for oral administration irradiated with the infrared light and a prepared reference mark match.

2. The method for determining the genuineness or spuriousness of a pharmaceutical composition for oral administration according to claim 1, wherein the infrared light may have a wavelength within a wavelength band range which includes at least a part of the range of 700 nm or more and 900 nm or less.

3. The method for determining the genuineness or spuriousness of a pharmaceutical composition for oral administration according to claim 1, wherein the wavelength band range of infrared light is a range of 900 nm or more.

4. The method for determining the genuineness or spuriousness of a pharmaceutical composition for oral administration according to any one of claims 1 to 3, wherein the pharmaceutical composition for oral administration includes titanium oxide and the mark including the edible substance which is visually recognizable under infrared irradiation is marked by laser irradiation.

5. The method for determining the genuineness or spuriousness of a pharmaceutical composition for oral administration according to any one of claims 1 to 3, wherein the mark including the edible substance which is visually recognizable under infrared irradiation is laser-printed using a pigment including yellow iron sesquioxide.

6. The method for determining the genuineness or spuriousness of a pharmaceutical composition for oral administration according to any one of claims 1 to 3, wherein the mark including the edible substance which is visually recognizable under infrared irradiation is printed using a pigment including at least one of edible charcoal and black triiron tetraoxide.

7. The method for determining the genuineness or spuriousness of a pharmaceutical composition for oral administration according to claim 2, wherein the mark including the edible substance which is visually recognizable under infrared irradiation is printed using a pigment including at least one selected from the group consisting of red iron oxide, sodium copper chlorophyllin, indigocarmine, and black titanium oxide.

8. The method for determining the genuineness or spuriousness of a pharmaceutical composition for oral administration according to any one of claims 1 to 7, wherein the mark including the edible substance which is not visually recognizable under infrared irradiation is printed using a dye.

9. The method for determining the genuineness or spuriousness of a pharmaceutical composition for oral administration according to claim 8, wherein the dye includes a tar dye.

10. The method for determining the genuineness or spuriousness of a pharmaceutical composition for oral administration according to claim 9, wherein the tar dye includes at least one selected from the group consisting of erythrosine, New Coccine, tartrazine, brilliant blue FCF, and sunset yellow FCF.

11. The method for determining the genuineness or spuriousness of a pharmaceutical composition for oral administration according to any one of claims 1 to 7, wherein the mark including the edible substance which is not visually recognizable under infrared irradiation is printed using a pigment including a tar dye aluminum lake.

12. The method for determining the genuineness or spuriousness of a pharmaceutical composition for oral administration according to claim 3, wherein the mark including the edible substance which is not visually recognizable under infrared irradiation is printed using a pigment including red iron oxide.

13. The method for determining the genuineness or spuriousness of a pharmaceutical composition for oral administration according to any one of claims 1 to 12, wherein, in the determining step, the reference mark corresponds to an identifying number or an identifying code for the pharmaceutical composition for oral administration.

14. The method for determining the genuineness or spuriousness of a pharmaceutical composition for oral administration according to claim 13, wherein the identifying number or the identifying code is marked on the pharmaceutical composition for oral administration.

15. The method for determining the genuineness or spuriousness of a pharmaceutical composition for oral administration according to any one of claims 1 to 14, wherein the pharmaceutical composition for oral administration is in a tablet form or a capsule form.

16. The method for determining the genuineness or spuriousness of a pharmaceutical composition for oral administration according to claim 15, wherein the pharmaceutical composition for oral administration is in a tablet form.

17. A pharmaceutical composition for oral administration marked with a mark including an edible substance which is visually recognizable under white light irradiation as well as under infrared irradiation and a mark including an edible substance which is visually recognizable under white light irradiation and is not visually recognizable under infrared irradiation, wherein the pharmaceutical composition for oral administration is able to be authenticated by determining whether the visually recognizable mark visually recognized on the pharmaceutical composition for oral administration and a prepared reference mark match.

18. A system for determining the genuineness or spuriousness of a pharmaceutical composition for oral administration, comprising
an irradiating device configured to irradiate a pharmaceutical composition for oral administration with infrared light, the pharmaceutical composition for oral administration being marked with a mark including an edible substance which is visually recognizable under white light irradiation as well as under infrared irradiation and a mark including an edible substance which is visually recognizable under white light irradiation and is not visually recognizable under infrared irradiation, and
a determining unit configured to determine whether the visually recognizable mark visually recognized on the pharmaceutical composition for oral administration irradiated with the infrared light and a prepared reference mark match.
